(19) Europäisches Patentamt
European Patent Office
Office européen des brevets

(11) EP 3 736 284 A1

# (12) EUROPEAN PATENT APPLICATION

(43) Date of publication:
**11.11.2020 Bulletin 2020/46**

(51) Int Cl.:
***C07K 14/705*** (2006.01) ***A61K 38/00*** (2006.01)

(21) Application number: **19382335.8**

(22) Date of filing: **06.05.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Iproteos S.L**
  **08028 Barcelona (ES)**
- **Consejo Superior De Investigaciones Científicas**
  **28006 Madrid (ES)**

(72) Inventors:
- **TARRAGÓ CLUA, María Teresa**
  **E- 08028 Barcelona (ES)**
- **PRADES COSANO, Roger**
  **E- 08028 Barcelona (ES)**
- **PALLARA, Chiara**
  **E- 08028 Barcelona (ES)**
- **LLORENTE CORTÉS, Vicenta**
  **E-08036 Barcelona (ES)**

(74) Representative: **ABG Intellectual Property Law, S.L.**
**Avenida de Burgos, 16D**
**Edificio Euromor**
**28036 Madrid (ES)**

## (54) VASCULAR CHOLESTEROL INHIBITORS AND USE THEREOF

(57) The present invention relates to compounds having pharmacological activity in the treatment of vascular cholesterol accumulation, plasma low-density lipoproteins (LDL) abnormal aggregation and/or inhibition or reduction of the formation of VSMC foam cells, processes for their preparation, pharmaceutical compositions comprising them, and their use in therapy and/or prophylaxis of conditions wherein decrease of vascular cholesterol accumulation, inhibition of LDL aggregation, inhibition or reduction of the formation of VSMC foam cells and/or prevention of aggregated LDL (agLDL) internalization is useful such as atherosclerosis, coronary artery disease, stroke, peripheral artery disease, angina pectoris, thrombosis, hyperlipidemia, hyperlipoproteinemia type II, familial hypercholesterolemia, familial combined hyperlipidemia, type II diabetes, hypothyroidism, Cushing's syndrome and obesity.

EP 3 736 284 A1

## SEQUENCE LISTING

```
<110>  Iproteos S.L.

<120>  VASCULAR CHOLESTEROL INHIBITORS AND USE THEREOF

<130>  P15762EP00

<160>  1

<170>  PatentIn version 3.5

<210>  1
<211>  14
<212>  PRT
<213>  Artificial Sequence

<220>
<223>  Peptide LP3

<400>  1

Gly Asp Asn Asp Ser Glu Asp Asn Ser Asp Glu Glu Asn Cys
1               5                   10
```

## Description

## FIELD OF THE INVENTION

The present invention relates to compounds having pharmacological activity in the treatment of vascular cholesterol accumulation and plasma low-density lipoproteins (LDL) abnormal aggregation, to processes of preparation of such compounds, to pharmaceutical compositions comprising them and their use in therapy and/or prophylaxis of conditions wherein decrease of vascular cholesterol accumulation, inhibition of LDL aggregation and/or prevention of aggregated LDL (agLDL) internalization is useful, such as atherosclerosis and all the atherosclerotic cardiovascular diseases (ASCVD) (e.g., coronary artery disease, stroke, peripheral artery disease, angina pectoris, thrombosis) as well as hypercolesterolemic conditions and/or abnormalities in lipoprotein metabolism (e.g. hyperlipidemia, hyperlipoproteinemia type II, familial hypercholesterolemia, familial combined hyperlipidemia, type II diabetes, hypothyroidism, Cushing's syndrome, obesity).

## BACKGROUND

Cardiovascular (CV) disease is the leading cause of mortality worldwide, causing about 31.4% of deaths in 2012. (World Health Organization: Health statistics and information systems. Cause-specific mortality. Glob Summ estimates for 2000-2012. Available at: "http://www.who.int/healthinfo/global_burden_disease/estimates/en/index1.html" Accessed January 2, 2015.) Data from 2010 demonstrate that CV disease accounted for 31.9% of US deaths, with ischemic heart disease and stroke leading to the vast majority (total 27.6%; 21.1%, and 6.5%, respectively) and generated a resulting global cost estimate of $863 billion in 2010, with a 22% increase expected by 2030. (Bloom, D.E., Cafiero, E.T., Jané-Llopis, E., Abrahams-Gessel, S., Bloom, L.R., Fathima, S., Feigl, A.B., Gaziano, T., Mowafi, M., Pandya, A., Prettner, K., Rosenberg, L., Seligman, B., Stein, A.Z., & Weinstein, C. (2011) The Global Economic Burden of Noncommunicable Diseases. Geneva: World Economic Forum (http://www3.weforum.org/docs/WEF_Harvard_HE_GlobalEconomicBurdenNonCom municableDiseases_2011.pdf).

A large, worldwide study demonstrated that among all modifiable risk factors, abnormal lipid levels were associated with the highest population attributable risk (approximately 50%) for the occurrence of myocardial infarction (MI) (Yusuf S et al. Lancet. 2004 Sep 11-17;364(9438):937-52 [PMID 15364185]). Similarly, the Framingham heart study showed that cardiovascular risk positively correlates with blood levels of LDL-cholesterol and inversely with HDL-cholesterol (Castelli WP et al. Ann Epidemiol. 1992 Jan-Mar;2(1-2):23-8 [PMID 1342260] and Gordon T et al. Am J Med. 1977 May;62(5):707-14 [PMID 193398]. Accordingly, in western countries, lifestyle interventions and evidence-based therapies, including those focused on hypercholesterolemia, have led to a reduction in CV risk at a population level. In a series of studies covering the 1980 to 2010 time period in the United States, Canada and Europe, it was estimated that 19%-46% of the total reduction in the rate of coronary heart disease (CHD) mortality was explained by a reduction in total cholesterol levels attributed to lifestyle changes and pharmacological treatment. (Björck L. et al. Eur Heart J. 2009 May;30(9):1046-56 [PMID 19141562], Bandosz P. et al. BMJ. 2012 Jan 25;344:d8136. [PMID 22279114], Wijeysundera HC. et al. JAMA. 2010 May 12;303(18):1841-7 [PMID 20460623], Flores-Mateo G. Rev Esp Cardiol. 2011 Nov;64(11):988-96 [PMDI 21962958], Hughes J. et al. Eur J Prev Cardiol. 2013 Apr;20(2):310-21 [PMID 22403395], Ford ES. et al. N Engl J Med. 2007 Jun 7;356(23):2388-98 [PMID 17554120], Aspelund T. et al. PLoS One. 2010 Nov 12;5(11):e13957 [PMID 21103050], Palmieri L. et al. Am J Public Health. 2010 Apr;100(4):684-92 [PMID 19608958], Bajekal M et al. PLoS Med. 2012;9(6):e1001237 [PMID 22719232] and Hotchkiss JW et al. BMJ. 2014 Feb 6;348:g1088 [PMID 24503058]).

Circulating blood cholesterol is mainly carried by low density lipoprotein (LDL) particles, which are thus key players in cholesterol transfer and metabolism from liver to any body cell (Hevonoja T. et al. Biochim Biophys Acta. 2000 Nov 15;1488(3):189-210 [PMID 11082530]). Circulating LDL particles are able to penetrate the endothelium of the arterial walls and became oxidized and aggregated, promote inflammation, and drive injury to the overlying endothelium and surrounding smooth muscle cells (Ross R. et al. N Engl J Med. 1999 Jan 14;340(2):115-26 [PMID 9887164]).

Persistent elevation in circulating LDL-cholesterol has been directly linked to alterations in the endothelium permeability that in turn promotes LDL influx into the arterial intima (Guretzki HJ. et al. Atherosclerosis. 1994 May;107(1):15-24 [PMID 7945555]). For example, LDL receptor-deficient mice (unable to clear LDL from the circulation) have elevated LDL-cholesterol and consequently develop severe atherosclerosis (Véniant MM. et al. J Clin Invest. 2000 Dec;106(12):1501-10 [PMID 11120757]). Conversely mice with virtually no LDL-cholesterol do not develop atherosclerosis regardless of diet and other heart disease risk factors (Lieu HD. et al. Circulation. 2003 Mar 11;107(9):1315-21 [PMID 12628954]). Epidemiological investigations have validated LDL-cholesterol blood level as an independent predictor of CV risk. According to the Framingham Heart Study, people exhibiting LDL-cholesterol level higher than 160mg/dL were more likely to develop clinically significant CHD compared to a reference population with LDL-cholesterol lower than 130mg/dL. (Wilson PW. et al. Circulation. 1998 May 12;97(18):1837-47 [PMID 9603539]). Similarly, in the Atherosclerosis Risk in Communities (ARIC) study was demonstrated that the risk of an incident CHD event was elevated by approximately 40% for every 39 mg/dL incremental increase in LDL-cholesterol (Sharrett AR. et al. Circulation. 2001 Sep 4;104(10):1108-13 [PMID 11535564]).

Together with abnormal LDL-cholesterol levels, alternative criteria related to the atherogenic lipoproteins have been demonstrated to be equally relevant CV risk factors. For example, it has been demonstrated that at identical LDL-cholesterol levels, measurement of the number of circulating LDL particles has important prognostic value since higher number of smaller LDL particles is associated with higher CV risk (Otvos JD. et al. J Clin Lipidol. 2011 Mar-Apr;5(2):105-13. doi: 10.1016/j.jacl.2011.02.001 [PMID 21392724]). Indeed, in one study of approximately 7000 participants without CV disease at baseline, LDL-attributable atherosclerotic risk was better indicated by LDL particle number when discordant with LDL-cholesterol levels (Otvos JD. et al. J Clin Lipidol. 2011 Mar-Apr;5(2):105-13. doi: 10.1016/j.jacl.2011.02.001 [PMID 21392724]). Similarly, pathological conditions exhibiting normal LDL cholesterol levels but small and dense LDL particles (such as in familial combined hyperlipidemia, type II diabetes and abdominal obesity) (Carr MC. et al. J Clin Endocrinol Metab. 2004 Jun;89(6):2601-7 [PMID 15181030]) have been found to be associated with an increased risk of myocardial infarction (Austin MA. et al. JAMA. 1988 Oct 7;260(13):1917-21[PMID 3418853], Stampfer MJ. et al. JAMA. 1996 Sep 18;276(11):882-8 [PMID 8782637], Lamarche B. et al. Circulation. 1997 Jan 7;95(1):69-75 [PMID 8994419] and worsened CHD severity (Tornvall P. et al. Atherosclerosis. 1991 Sep;90(1):67-80 [PMID 1799399], Campos H. et al. Arterioscler Thromb. 1992 Feb;12(2):187-95 [PMID 1543692], Gardner CD. et al. JAMA. 1996 Sep 18;276(11):875-81 [PMID 8782636]). This relation may be due to the enhanced delivery of cholesterol to an atheroma by greater numbers of small and dense LDL particles (Tabas I. et al. Circulation. 2007 Oct 16;116(16):1832-44 [PMID 17938300]), higher susceptibility to oxidation and aggregation, reduced affinity to the LDL receptor (Berneis KK. et al. J Lipid Res. 2002 Sep;43(9):1363-79 [PMID 12235168]), higher affinity to intimal proteoglycans (Anber V. et al. Arterioscler Thromb Vasc Biol. 1997 Nov;17(11):2507-14 [PMID 9409221]) and higher tendency to form LDL aggregates (Hurt-Camejo E. et al. Arthritis Rheum. 2001 Dec;44(12):2761-7 [PMID: 11762936]; Sartipy P. et al. J Biol Chem. 1999 Sep 3;274(36):25913-20 [PMID: 10464335]; Camejo G. et al. Atherosclerosis. 1998 Aug;139(2):205-22 [PMID: 9712326]).

The accumulation of LDL-cholesterol in the arterial intima is a critical step in vascular cholesteryl ester (CE) deposition since increases the tendency of atherosclerotic plaque to rupture thus triggering the thrombotic process and the development of ischemic cardiomyopathy (Aikawa M. et al. Cardiovasc Pathol. 2004 May-Jun;13(3):125-38 [PMID 15081469], Mauriello A. et al. Atherosclerosis. 2010 Feb;208(2):572-80 [PMID 19683236], Puri R. et al. Arterioscler Thromb Vasc Biol. 2016 Nov;36(11):2220-2228 [PMID 27515380]. Cholesteryl esters (CE) in the atherosclerotic plaques are deposited both extra- and intra-cellularly. Extracellular deposition of LDL-cholesteryl esters, a crucial initiating event in atherosclerosis (Skålén K. et al. Nature. 2002 Jun 13;417(6890):750-4 [PMID 12066187], Tabas I. et al. Circulation. 2007 Oct 16;116(16):1832-44 [PMID 17938300]), is mediated by the proteoglycans that conform the extracellular matrix of the arterial intima. The electrostatic interactions between proteoglycans and LDL and the proteolytic degradation of LDL, are steps extremely facilitated in the arterial intima and both promote LDL retention and aggregation (Oörni K. et al. J Lipid Res. 2000 Nov;41(11):1703-14 [PMID 11060340], Oörni K. et al. Arterioscler Thromb Vasc Biol. 2005 Aug;25(8):1678-83 [PMID 15879301]).

LDL aggregation has been reported to be mainly modulated by two enzymes: sphingomyelinase (SMase), secreted by endothelial cells and macrophages (Marathe S. et al. J Biol Chem. 1998 Feb 13;273(7):4081-8 [PMID 9461601], Schissel SL. et al. J Biol Chem. 1996 Aug 2;271(31):18431-6 [PMID 8702487]), and phospholipase A2 (PLA2). Both SMase and PLA2 are crucial in the process of intimal LDL aggregation during atherogenesis (Aviram M. et al. Biochem Biophys Res Commun. 1992 May 29;185(1):465-72 [PMID 1599485], Oörni K. et al. J Biol Chem. 1998 Oct 30;273(44):29127-34 [PMID 9786921], Hakala JK. et al. Arterioscler Thromb Vasc Biol. 2001 Jun;21(6):1053-8 [PMID 11397719], Tabas I. et al. J Biol Chem. 1993 Sep 25;268(27):20419-32 [PMID 8376399]). AgLDL have been detected and isolated from atherosclerotic plaques in in vivo animal models and humans (Schissel SL. et al. J Clin Invest. 1996 Sep 15;98(6):1455-64 [PMID 8823312], Wyler von Ballmoos M. et al. Arterioscler Thromb Vasc Biol. 2006 Feb;26(2):359-64 [PMID 16322531]). Unlike native LDL (nLDL), agLDL are a potent inducer of massive intracellular CE accumulation in both macrophages (Khoo JC. et al. Arteriosclerosis. 1988 Jul-Aug;8(4):348-58 [PMID 3395271], Zhang WY. et al. J Biol Chem. 2000 Oct 20;275(42):33176-83 [PMID 10942782], Kruth HS. Curr Opin Lipidol. 2002 Oct;13(5):483-8 [PMID 12352011]) and human coronary vascular smooth muscle cells (hcVSMCs) (Llorente-Cortés V. et al. Arterioscler Thromb Vasc Biol. 1998 May;18(5):738-46 [PMID 9598832] http://atvb.ahajournals.org/content/20/6/1572 Llorente-Cortés V. et al. Arterioscler Thromb Vasc Biol. 2002 Mar 1;22(3):387-93 [PMID 11884279], Llorente-Cortés V. et al. Circulation. 2002 Dec 10;106(24):3104-10 [PMID 12473559]). It was recently demonstrated that in hcVSMC, AgLDL are avidly taken up through the low-density lipoprotein receptor-related protein 1 (LRP1) (Llorente-Cortés V. et al. Arterioscler Thromb Vasc Biol. 1998 May;18(5):738-46 [PMID 9598832], Llorente-Cortés V. et al. Arterioscler Thromb Vasc Biol. 2002 Mar 1;22(3):387-93 [PMID 11884279]. AgLDL internalization, in turn, induces LRP1 expression (Llorente-Cortés V. et al. Circulation. 2002 Dec 10;106(24):3104-10 [PMID 12473559], Costales P. et al. Atherosclerosis. 2010 Dec;213(2):458-68 [PMID 20980003], Llorente-Cortés V. et al. J Mol Biol. 2006 Jun 16;359(4):950-60 [PMID 16697011]), promoting a positive feedback loop that efficiently transforms hcVSMC into foam

cells. Foam cells are a crucial vascular component determining the susceptibility of atherosclerotic plaque to rupture. It has been also demonstrated that hcVSMC-foam cells synthesize and release high amounts of tissue factor, key for the prothrombotic transformation of the vascular wall and thus for the progression of atherosclerosis to thrombosis (Llorente-Cortés V. et al. Circulation. 2004 Jul 27;110(4):452-9 [PMID 15238452], Camino-López S. et al. Cardiovasc Res. 2007 Jan 1;73(1):208-16 [PMID 17141748], Camino-López S. et al. Thromb Haemost. 2009 Dec;7(12):2137-46 [PMID 19817993]). The relevance of this mechanism in atherosclerosis is evident since VSMC are the main cellular component of the vascular wall and more than 50% of the foam cells in human atherosclerotic plaques, previously regarded as monocyte-derived macrophages, originate from VSMC (Allahverdian S. et al. Circulation. 2014 Apr 15;129(15):1551-9 [PMID 24481950]). Taken together, these findings support that LRP1 plays a crucial role in the generation of hVSMC-derived foam cells through LRP1-mediated AgLDL uptake and cholesterol accumulation in the vasculature susceptible to develop atherosclerosis.

LRP1 is a key signalling protein and thus is involved in various biological processes, including lipoprotein metabolism, and diseases, such as atherosclerosis (Etique N. et al. Biomed Res Int. 2013;2013:152163 [PMID 23936774], Lillis AP. et al. J Thromb Haemost. 2005 Aug;3(8):1884-93 [PMID 16102056]). AgLDL is the first ligand reported to interact with a unique LRP1 domain, CR9, which seems to exclusively recognize agLDL. AgLDL are thus specifically recognized by the region Gly1127-Cys1140 (peptide LP3: H-GDNDSEDNSDEENC-$NH_2$ SEQID NO: 1) that spans the C-terminal half of domain CR9, which has been reported to be crucial for binding to AgLDL and its subsequent internalization in human VSMC (Costales P. et al. J Biol Chem. 2015 Jun 12;290(24):14852-65 [PMID 25918169]).

Currently the prevention of atherosclerosis and other CV diseases is mainly based on lipid-lowering agents (i.e., HMG-CoA reductase and PCSK9 inhibitors) that reduce blood cholesterol levels. Although this reduction of plasma cholesterol levels undoubtedly affects the amount of cholesterol that is retained and accumulates in the vascular wall of the coronary vessels, it was found to be not enough to avoid the risk and the mortality of CV events. Indeed, the benefit of lipid-lowering drugs, such as statin-based therapies is often not related to a sharp decrease in CV mortality (acute myocardial infarction and angina pectoris) in atherosclerosis patients (DuBroff R. et al. World J Cardiol. 2015 Jul 26;7(7):404-9 [PMID 26225201]).

These data clearly demonstrate the crucial need to block atherosclerotic process acting on mechanisms that take place specifically in the vascular wall, by controlling the AgLDL internalization in VSMC and/or modulating the inflammatory component of the atherosclerotic plaque.

Moreover, although statin-based therapy is generally well tolerated and highly effective in lowering blood cholesterol levels, it can be associated with various adverse events (e.g, intolerance, myalgia, myopathy, rhabdomyolysis, and diabetes mellitus, among others) (Toth PP. et al. Am J Cardiovasc Drugs. 2018 Jun;18(3):157-173 [PMID 29318532]). And still more, it is also demonstrated that it is generally related to an increased incidence of diabetes (Barylski M. et al. Curr Pharm Des. 2014;20(22):3657-64 [PMID 24040871]). Indeed, being exactly diabetic patients the ones with a higher incidence of atherosclerosis and cardiovascular pathology, the development of innovative drugs for the treatment of atherosclerosis in diabetic patient or with high susceptibility of diabetes development is becoming of paramount importance.

In this context, inhibiting vascular cholesterol accumulation by modulating not only LDL aggregation but also aggregated LDL internalization by vascular cells appear to be a promising therapeutical strategy in the treatment of cardiovascular disease. Moreover, the pivotal role of the lipoprotein receptor LRP1, an in particular of the C-terminal half of CR9 domain (peptide LP3) in agLDL binding and internalization by hcVSMC, suggest that compounds derived from LP3 sequence would be highly promising compounds which would decrease LDL aggregation, agLDL internalization and VSMC-foam cell formation.

In patients with ischemic stroke, an elevation of electronegative LDL has been reported (Podrez EA. et al. Blood.2016 Mar 10;127(10):1221-2. [PMID: 26965920]). Electronegative LDL is a fraction of smaller LDL with higher tendency for LDL aggregation (Bancells C. et al. J Lipid Res. 2009 Mar;50(3):446-55. Bancells C. et al. Biochemistry. 2008 Aug 5;47(31):8186-94; Bancells C. et al. J Biol Chem. 2010 Oct 15;285(42):32425-35) with predictive value in cardiovascular risk (Ivanova EA et al. Vasc Health Risk Manag. 2015 Aug 28;11:525-32).

In peripheral artery disease (PAD), atherogenic dyslipemia, characterized by increased number of small LDL particle with higher tendency for aggregation, is the primary lipid driver for PAD risk (Aday AW. et al. Lipoprotein Particle Profiles, Standard Lipids, and Peripheral Artery Disease Incidence - Prospective Data from the Women's Health Study (Circulation. 2018, in press).

Type III hyperlipoproteinemia (HLP), or dysbetalipoproteinemia, and familial hypercholesterolemia (HF) are genetic disorders of lipid metabolism that predisposes affected subjects to the premature development of atherosclerosis. These disorders are characterized by elevated plasma cholesterol (Mahley RW et al. J Lipid Res. 1999 Nov;40(11):1933-49; Santos RD et al. Lancet Diabetes Endocrinol. 2016 Oct;4(10):850-61. doi: 10.1016/S2213-8587(16)30041-9. Epub 2016 May 27. Review. PMID: 27246162; Sturm AC, et al. J Am Coll Cardiol. 2018 Aug 7;72(6):662-680. PMID:30071997).

Familial combined hiperlipidemia (FCHL), is a common primary dyslipidemia that courses with hypercholesterolemia or high LDL colesterol levels in which apolipoprotein B containing particle composition is abnormal and interferes with LDL-C estimation (Mehta R, et al. Atherosclerosis. 2018 Oct;277:204-210. PMID: 29970255).

Type 2 diabetic (T2DM) and obese patients are characterized by atherogenic dyslipemia, characterized by increased number of small LDL particle with higher tendency for aggregation (Millán J et al. Med Clin (Bare). 2013 Nov 16;141(10):430-6. PMID: 23246165; Athyros VG, et al. Hormones (Athens). 2018 Mar;17(1):61-67. PMID: 29858856; Iqbal J, et al. Curr Diabetes Rev. 2018;14(5):427-433. doi: PMID:28677496; Gerber PA, Nikolic D, Rizzo M. Small, dense LDL: an update. Curr Opin Cardiol. 2017 Jul;32(4):454-459. PMID: 28426445; Sartipy P, et al. J Biol Chem. 1999 Sep3;274(36):25913-20. PubMed PMID: 10464335; Camejo G, et al. Atheroscler Suppl. 2002 May;3(1):3-9. Review. PMID: 12044579). Therefore, this type of dyslipemia is likely one of the primary causes of the higher prevalence of atherosclerosis in this group of patients.

Hypothyroidism negatively affects lipid metabolism leading to hypercholesterolemia which progressively increases the risk for cardiovascular disease and, potentially, mortality. Hypercholesterolemia in hypothyroidism is mainly due to a reduction in LDL receptor activity (Jayasingh IA et al. J Family Med Prim Care. 2016 Oct-Dec;5(4):809-816. doi: 10.4103/2249-4863.201177; Duntas LH et al, Front Endocrinol (Lausanne) 2018; 9: 511.PMCID: PMC6129606).

Cushing's syndrome (CS), including visceral obesity, dyslipidemia, hypertension and diabetes among its many manifestations, is "a model" of metabolic syndrome. Dyslipidemia is a common finding in CS as a consequence of GC-related increased lipolysis, lipogenesis and adipogenesis. Ferraù F, et al. Front Horm Res. 2018;49:85-103. doi: 10.1159/000486002. PMID: 29894989.

## BRIEF DESCRIPTION OF THE INVENTION

The inventors have successfully found that a family of compounds of formula (I) are capable of efficiently preventing LDL aggregation and VSMC-foam cell formation. These properties make the compounds of the present invention ideal candidates for the use in the therapy of atherosclerosis and all the atherosclerotic cardiovascular diseases (ASCVD) (e.g., coronary artery disease, stroke, peripheral artery disease, angina pectoris, thrombosis) as well as hypercolesterolemic conditions and/or abnormalities in lipoprotein metabolism (e.g. hyperlipidemia, hyperlipoproteinemia type II, familial hypercholesterolemia, familial combined hyperlipidemia, type II diabetes, hypothyroidism, Cushing's syndrome, obesity).

"LDL aggregation" refers to a process characterized in that Low-Density Lipoprotein (LDL) particles circulating in the blood bind to and are retained by extracellular matrix components such as proteoglycans in the arterial wall. The retained lipoproteins subsequently undergo various modifications, including oxidation, lipolysis, and proteolysis by resident hydrolytic and oxidative enzymes. These modifications cause LDL fusion that further augments LDL retention in the arterial wall, triggering a cascade of inflammatory and apoptotic responses that contribute to atherogenesis. Thus, the compounds of the invention prevent this interaction of LDL particles with the arteria wall and/or its retention.

"LDL aggregation" can be measured by LDL turbidimetry (absorbance at 405 nm) after LDL isolation from serum/plasma (standardized method to measure this process in patients has been reported in Ruuth M et al. Eur Heart Journal 2018) thus "preventing LDL aggregation" will reduce or impede the measured levels.

"VSMC-foam cell formation" refers to "Vascular smooth muscle cell (VSMC) foam cell formation"; these "VSMC-foam cells" are fat-laden vascular smooth muscle cells that have developed in response to an initial lipid injury and acquire a synthetic and proliferative phenotype and lose several markers of their physiological contractile function. Foam VSMCs can evolve either towards cell death, promoting intimal proliferation of adjacent VSMCs and plaque calcification, hence participating in atherome progression, or towards more complex and tissue-integrated responses. Lipid LDL contents, such as free cholesterol and cholesterol crystal or metabolized phospholipids can be accumulated within cells and released into the extracellular space reinforcing the lesion progression.

LDL aggregation and VSMC-foam cell formation are two events related with atherosclerosis, a disease in which the inside of an artery narrows due to the buildup of an atherome, also known as atheroatheroma or atheromatous plaque ("plaque"). Atherosclerosis is initiated by inflammatory processes in the endothelial cells of the vessel wall associated with retained low-density lipoprotein (LDL) particles. The ensuing inflammation leads to formation of atheromatous plaques in the arterial tunica intima. Macrophages recruited to the inflammation site become foam cells and platelets encourage the migration and proliferation of smooth muscle cells, which in turn ingest lipids, become replaced by collagen and transform into foam cells themselves (VSMC-foam cells). A protective fibrous cap normally forms between the fatty deposits and the artery lining (the intima), thus narrowing the lumen and stiffening the arterial wall.

Atherosclerotic cardiovascular diseases (ASCVD) are characterised by the buildup of atheromas in arteries, the list of ASCV diseases includes coronary artery disease, stroke, peripheral artery disease, angina pectoris and thrombosis. Some hypercolesterolemic conditions and/or abnormalities in lipoprotein metabolism also prone to develop these arterial atheromas such as hyperlipidemia, hyperlipoproteinemia type II, familial hypercholesterolemia, familial combined hyperlipidemia, type II diabetes, hypothyroidism, Cushing's syndrome, obesity.

Therefore, one aspect of the invention relates to compounds having the formula (I):

$$R^1\text{-}AA^1\text{-}AA^2\text{-}AA^3\text{-}AA^4\text{-}AA^5\text{-D-Glu-D-Asp-}AA^6\text{-}AA^7\text{-}AA^8\text{-D-Glu-D-Glu-}AA^9\text{-}NH_2 \quad (I)$$

wherein

$R^1$ is a $C_{2-4}$ acyl group,

$AA^1$ is either absent or is Gly

$AA^2$ is either absent or is D-Asp

$AA^3$ is either absent or is D-Asn

$AA^4$ is either absent or is D-Asp

$AA^5$ is selected from the group consisting of D-Ser and D-Ala

$AA^6$ is selected from the group consisting of D-Asn and D-Gln

$AA^7$ is selected from the group consisting of D-Ala, D-Arg, D-Lys and D-Ser

$AA^8$ is selected from the group consisting of D-Asp and D-Glu

$AA^9$ is selected from the group consisting of D-Ala, D-Arg, D-Asn, D-Gln, D-Leu and D-Trp

or a salt thereof.

Another aspect of this invention refers to processes for the preparation of a compound of formula (I) as defined above or a pharmaceutically acceptable salt thereof.

Another aspect of this invention refers to pharmaceutical compositions comprising a compound of formula (I) as defined above, or a pharmaceutically acceptable salt thereof and a pharmaceutically acceptable carrier, adjuvant or vehicle.

Another aspect of this invention refers to a compound of formula (I) as defined above, or a pharmaceutically acceptable salt thereof, for use as a medicament, particularly for the prevention and/or treatment of conditions wherein decrease of vascular cholesterol accumulation, inhibition of LDL aggregation; prevention of aggregated LDL (agLDL) internalization and/or inhibition or reduction of the formation of VSMC foam cells is useful, in particular for the prevention or treatment of atherosclerosis and all the atherosclerotic cardiovascular diseases (ASCVD) (such as, coronary artery disease, stroke, peripheral artery disease, angina pectoris, thrombosis) as well as hypercolesterolemic conditions and/or abnormalities in lipoprotein metabolism (e.g. hyperlipidemia, hyperlipoproteinemia type II, familial hypercholesterolemia, familial combined hyperlipidemia, type II diabetes, hypothyroidism, Cushing's syndrome, obesity).

Another aspect of this invention refers to a method for the treatment or prophylaxis of conditions wherein decrease of vascular cholesterol accumulation, inhibition of LDL aggregation; prevention of aggregated LDL (agLDL) internalization and/or inhibition or reduction of the formation of VSMC foam cells is useful, in particular for the prevention or treatment of atherosclerosis and all the atherosclerotic cardiovascular diseases (ASCVD) (e.g., coronary artery disease, stroke, peripheral artery disease, angina pectoris, thrombosis) as well as hypercolesterolemic conditions and/or abnormalities in lipoprotein metabolism (e.g. hyperlipidemia, hyperlipoproteinemia type II, familial hypercholesterolemia, familial combined hyperlipidemia, type II diabetes, hypothyroidism, Cushing's syndrome, obesity) wherein a therapeutic amount of a compound of formula (I) as defined above, or a pharmaceutically acceptable salt, prodrug or solvate therefore, is administered to a patient in need of said treatment.

Another aspect of this invention refers to the use of a compound of formula (I) as defined above, or a pharmaceutically acceptable salt thereof, for the preparation of a medicament, particularly for the prevention and/or treatment of conditions wherein decrease of vascular cholesterol accumulation, inhibition of LDL aggregation; prevention of aggregated LDL (agLDL) internalization and/or inhibition or reduction of the formation of VSMC foam cells is useful, in particular for the prevention or treatment of a disease selected from the group consisting of atherosclerosis and all the atherosclerotic cardiovascular diseases (ASCVD) (e.g., coronary artery disease, stroke, peripheral artery disease, angina pectoris, thrombosis) as well as hypercolesterolemic conditions and/or abnormalities in lipoprotein metabolism (e.g. hyperlipidemia, hyperlipoproteinemia type II, familial hypercholesterolemia, familial combined hyperlipidemia, type II diabetes, hypothyroidism, Cushing's syndrome, obesity).

These aspects and preferred embodiments thereof are additionally also defined in the claims.

## DETAILED DESCRIPTION OF THE INVENTION

In the context of the present invention some abbreviations and acronyms have been use and their meanings are provided below:

| | |
|---|---|
| Ac | Acetyl |
| agLDL | Aggregated LDL |
| D-Ala | D-alanine |
| Alloc | Allyloxycarbonyl |
| ApoB | Apolipoportein B |
| D-Arg | D-arginine |
| D-Asn | D-asparagine |
| D-Asp | D-aspartic acid |
| ASCVD | Atherosclerotic cardiovascular diseases |
| eq | Equivalent |
| Boc | Butyloxycarbonyl |
| °C | Celsius degree |
| CE | Cholesteryl esters |
| CV | Cardiovascular |
| DBU | 1,8-Diazabicyclo[5.4.0]undec-7-ene |
| BSA | Bovine serum albumin |
| DCM | Dichloromethane |
| DIC | N,N'-Diisopropylcarbodiimide |
| DIEA | N,N-diisopropylethylamine |
| DMF | Dimethylformamide |
| DMSO | Dimethyl sulfoxide |
| EDTA | Ethylendiaminetetraacetic acid |
| FC | Free cholesterol |
| Fmoc | Fluorenylmethyloxycarbonyl |
| D-Gln | D-glutamine |
| D-Glu | D-glutamic acid |
| Gly | glycine |
| HMG-CoA | β-Hydroxy β-methylglutaryl-Coenzyme A |
| HPLC | High Performance Liquid Chromatography |
| HPLC-MS | High Performance Liquid Chromatography coupled to mass spectometer |
| hVSMC | Human coronary vascular smooth muscle cells |
| D-Leu | D-leucine |
| LDL | Low-density lipoprotein |
| D-Lys | D-lysine |
| M | Molar |
| MeOH | Methanol |
| OtBu | tert-butyl ester |
| Oxyma pure | Ethyl cyano(hydroxyimino)acetate |
| Pbf | 2,2,4,6,7-pentamethyldihydrobenzofuran-5-sulfonyl |
| PBS | Phosphate buffered saline |
| PCSK9 | Proprotein convertase subtilisin/kexin type 9 |
| PLA2 | Phospholipase A2 |
| rpm | Revolutions per minute |
| D-Ser | D-serine |
| SMASE | Sphingomyelinase |
| TFA | Trifluoroacetic acid |
| TIS | Triisopropylsilane |
| Tris | Tris(hydroxymethyl)aminomethane |
| D-Trp | D-tryptophan |
| Trt | Trityl |
| VLDL | Very low density lipoprotein |

In the present invention the notation -$AA^n$- and the notation -D-xxx- wherein "xxx" stands for the 3-letters amino

acid abbreviation code, are used to designate an amino acid whose amino group is devoid of one of its hydrogen atoms (H) and its carboxylic group is devoid of the rest OH. As a mere example -D-Arg- is used to represent D-Arginine whose $\alpha$-amino group lacks a hydrogen and its $\alpha$-carboxylic group lacks the rest OH.

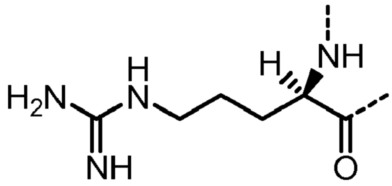

The term "salt" must be understood as any form of an active compound used in accordance with this invention in which said compound is in ionic form or is charged and coupled (associated) to a counter-ion (a cation or anion) either in solid form or in solution. This definition also includes quaternary ammonium salts. The definition includes in particular physiologically acceptable salts; this term must be understood as equivalent to "pharmacologically acceptable salts" or "pharmaceutically acceptable salts".

The term "pharmaceutically acceptable salts" in the context of this invention means any salt that is tolerated physiologically (normally meaning that it is not toxic, particularly, as a result of the counter-ion) when used in an appropriate manner for the treatment, applied or used, particularly, in humans and/or mammals. These physiologically acceptable salts may be formed with cations associated to negatively charged groups of the peptides or anions associated with positively charged groups of the peptides. Said charged groups may be the amino terminal ($-NH_2$) group when $R^1$ is hydrogen or lateral side-chain groups such as the carboxylic lateral group of aspartic acid and glutamic acid, the amino lateral side-chain group of lysine or the guanidino lateral side-chain group of arginine. This definition specifically includes in the context of this invention a salt formed by a physiologically tolerated acid, i.e. salts of a specific active compound with physiologically tolerated organic or inorganic acids- particularly when used on humans and/or mammals.

Pharmaceutically acceptable acids include inorganic acids, such as hydrochloric, sulphuric, phosphoric, diphosphoric, hydrobromic, hydroiodic and nitrate acids, and organic acids, such as citric, maleic, malic, mandelic, ascorbic, oxalic, succinic, tartaric, acetic, methanesulfonic, ethanesulfonic, benzenesulfonic and p-toluenosulfonic acids. Pharmaceutically acceptable bases include hydroxides of alkali metals (e.g. sodium or potassium), alkaline-earth metals (for example, calcium or magnesium) and organic bases (for example, alkylamines, arylalkyilamines and heterocyclic amines).

Other preferred salts according to the invention are quaternary ammonium compounds in which an equivalent of an anion (X-) is associated with the positive charge of the N atom. X- may be an anion of diverse mineral acids such as for example, chloride, bromide, iodide, sulfate, nitrate, phosphate, or an anion of an organic acid, such as acetate, maleate, fumarate, citrate, oxalate, succinate, tartrate, malate, mandelate, trifluoracetate, methanesulfonate and p-toluenesulfonate. X- is preferably an anion selected from chloride, bromide, iodide, sulfate, nitrate, acetate, maleate, oxalate, succinate and trifluoracetate. More preferably X- is chloride, bromide, trifluoracetate or methanesulfonate.

Unless otherwise stated, the compounds of the invention are also meant to include isotopically-labelled forms i.e. compounds which differ only in the presence of one or more isotopically-enriched atoms. For example, compounds having the present structures except for the replacement of at least one hydrogen atom by a deuterium or tritium, or the replacement of at least one carbon by $^{13}C$- or $^{14}C$-enriched carbon, or the replacement of at least one nitrogen by $^{15}N$-enriched nitrogen are within the scope of this invention.

The compounds of formula (I), or their salts or solvates (such as hydrates) are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.

As noted previously, the term "pharmaceutically acceptable salts, solvates such as hydrates, prodrugs" refers to any salt, solvate, or any other compound which, upon administration to the recipient, is capable of providing (directly or indirectly) a compound as described herein. However, it will be appreciated that non-pharmaceutically acceptable salts, solvates such as hydrates and prodrugs of the compounds of formula (I) also fall within the scope of the invention since those may be useful in the preparation of pharmaceutically acceptable salts, solvates such as hydrates and prodrugs of said compounds. The preparation of salts, solvates and prodrugs can be carried out by methods known in the art.

In one embodiment of the present invention in the compounds of formula (I) $R^1$ is acetyl.

In another embodiment of the present invention in the compounds of formula (I) the rest $AA^1$ is absent.

In another embodiment of the present invention in the compounds of formula (I) $AA^2$ is absent.

In another embodiment of the present invention in the compounds of formula (I) $AA^3$ is absent.

In another embodiment of the present invention in the compounds of formula (I) the rest wherein $-AA^1-AA^2-AA^3-AA^4-AA^5-$ is either absent or is selected from the group consisting of -Gly-D-Asp-D-Asn-D-Asp-D-Ser, -D-Asp-D-Asn-D-Asp-D-Ser- and -D-Asn-D-Asp-D-Ser-, -D-Asp-D-Ser and -D-Asp-D-Ala-.

In another embodiment of the present invention in the compounds of formula (I) $AA^5$ is D-Ser.

In another embodiment of the present invention in the compounds of formula (I) the rest $-AA^6-AA^7-AA^8-$ is selected from the group consisting of -D-Asn-D-Ser-D-Asp-, -D-Asn-D-Ala-D-Asp-, -D-Gln-D-Ser-D-Glu-, -D-Gln-D-Ala-D-Glu-, -D-Asn-D-Arg-D-Asp- and -D-Asn-D-Lys-D-Asp-.

In still another embodiment of the present invention in the compounds of formula (I) the rest $-AA^6-AA^7-AA^8-$ is selected from the group consisting of -D-Asn-D-Ser-D-Asp-.

In another embodiment of the present invention in the compounds of formula (I) wherein $-AA^9-$ is selected from the group consisting of D-Ala, D-Asn, D-Gln, D-Ala, D-Arg, D-Leu and D-Trp.

In still another embodiment of the present invention in the compounds of formula (I) wherein $-AA^9-$ is selected from the group consisting of D-Ala and D-Asn.

Particular individual compounds of the invention falling under formula (I) include the compounds listed below:

- Ac-Gly-D-Asp-D-Asn-D-Asp-D-Ser-D-Glu-D-Asp-D-Asn-D-Ser-D-Asp-D-Glu-D-Glu-D-Asn-$NH_2$
- Ac-D-Ser-D-Glu-D-Asp-D-Asn-D-Ser-D-Asp-D-Glu-D-Glu-D-Asn-$NH_2$
- Ac-D-Ser-D-Glu-D-Asp-D-Asn-D-Ala-D-Asp-D-Glu-D-Glu-D-Ala-$NH_2$
- Ac-D-Ser-D-Glu-D-Asp-D-Gln-D-Ser-D-Glu-D-Glu-D-Glu-D-Gln-$NH_2$
- Ac-D-Ser-D-Glu-D-Asp-D-Gln-D-Ala-D-Glu-D-Glu-D-Glu-D-Ala-$NH_2$
- Ac-D-Ser-D-Glu-D-Asp-D-Asn-D-Arg-D-Asp-D-Glu-D-Glu-D-Arg-$NH_2$
- Ac-D-Ser-D-Glu-D-Asp-D-Asn-D-Arg-D-Asp-D-Glu-D-Glu-D-Leu-$NH_2$
- Ac-D-Ser-D-Glu-D-Asp-D-Asn-D-Arg-D-Asp-D-Glu-D-Glu-D-Trp-$NH_2$
- Ac-D-Ser-D-Glu-D-Asp-D-Asn-D-Lys-D-Asp-D-Glu-D-Glu-D-Asn-$NH_2$
- Ac-D-Asp-D-Asn-D-Asp-D-Ser-D-Glu-D-Asp-D-Asn-D-Ser-D-Asp-D-Glu-D-Glu-D-Asn-$NH_2$
- Ac-D-Asn-D-Asp-D-Ser-D-Glu-D-Asp-D-Asn-D-Ser-D-Asp-D-Glu-D-Glu-D-Asn-$NH_2$
- Ac-D-Asp-D-Ser-D-Glu-D-Asp-D-Asn-D-Ser-D-Asp-D-Glu-D-Glu-D-Asn-$NH_2$
- Ac-D-Asp-D-Asn-D-Asp-D-Ser-D-Glu-D-Asp-D-Asn-D-Ser-D-Asp-D-Glu-D-Glu-D-Ala-$NH_2$
- Ac-D-Asn-D-Asp-D-Ser-D-Glu-D-Asp-D-Asn-D-Ser-D-Asp-D-Glu-D-Glu-D-Ala-$NH_2$
- Ac-D-Asp-D-Ser-D-Glu-D-Asp-D-Asn-D-Ser-D-Asp-D-Glu-D-Glu-D-Ala-$NH_2$
- Ac-D-Asp-D-Ser-D-Glu-D-Asp-D-Asn-D-Ala-D-Asp-D-Glu-D-Glu-D-Ala-$NH_2$
- Ac-D-Asp-D-Ala-D-Glu-D-Asp-D-Asn-D-Ala-D-Asp-D-Glu-D-Glu-D-Ala-$NH_2$
- Ac-D-Asp-D-Ser-D-Glu-D-Asp-D-Asn-D-Lys-D-Asp-D-Glu-D-Glu-D-Asn-$NH_2$
- Ac-D-Asp-D-Ser-D-Glu-D-Asp-D-Asn-D-Lys-D-Asp-D-Glu-D-Glu-D-Ala-$NH_2$

or a pharmaceutically acceptable salt thereof.

The compounds of formula (I) defined above can be obtained by available synthetic procedures as illustrated by the following general scheme:

<u>DETAILED DESCRIPTION OF THE ROUTE OF SYNTHESIS OF COMPOUNDS OF FORMULA (I)</u>

A polymeric support (resin), suitable to be coupled to Fmoc-protected aminoacids (such as Nα-Fmoc-N-β-(Trt)-D-asparagine (Fmoc-D-Asn(Trt)-OH), Nα-Fmoc-D-alanine (Fmoc-D-Ala-OH), Nα-Fmoc-N-γ-(Trt)-D-glutamine (Fmoc-D-Gln(Trt)-OH), Nα-Fmoc-D-leucine (Fmoc-D-Leu-OH), Nα-Fmoc-N-in-Boc-D-tryptophan (Fmoc-D-Trp(Boc)-OH) and Nα-Fmoc-NG-(2,2,4,6,7-pentamethyldihydrobenzofuran-5-sulfonyl)-D-arginine (Fmoc-D-Arg(pbf)-OH)) through their carbonyl group and to yield upon cleavage a C-terminal amide group, such as H-Rink Amide-ChemMatrix® (I), is placed in syringe fitted with a polyethylene porous disk (reaction vessel).

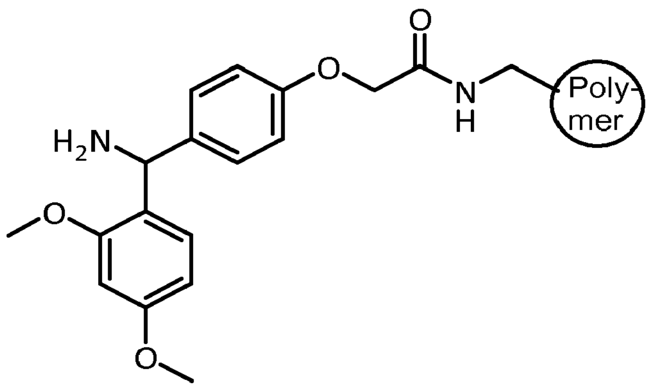

(I)

The resin is swelled by washes with appropriate organic solvents such as dichloromethane (DCM) and dimethylformamide (DMF).

Following swelling and preparation of the resin, the protected form of the first amino acid ($AA^9$) (i.e. a protected amino acid selected from the group consisting of Fmoc-D-Asn(Trt)-OH, Fmoc-D-Ala-OH, Fmoc-D-Glu(Trt)-OH, Fmoc-D-Leu-OH, Fmoc-D-Trp(Boc)-OH and Fmoc-D-Arg(pbf)-OH) of the peptide to be synthetized is attached to the resin through its carboxylic acid moiety using a coupling reagent such as N,N'-Diisopropylcarbodiimide (DIC) and the use of coupling additive such as Oxyma Pure in an appropriate organic solvent such as DMF. To perform the coupling, the protected amino acid is mixed with the coupling additive and with the coupling agent. The mixture is stirred or allowed to stand for a few minutes. Then, the mixture is added to the reaction vessel containing the swelled resin and the reaction mixture is intermittently stirred for 1-5 hours. After that, the solvents and unreacted reagents are removed by suction. The performance of the reaction is monitored using a calorimetric test suitable for the detection of amines on polymeric supports, such as the Kaiser test (E. Kaiser, Anal biochem. 1970), the coupling treatment is repeated if the coupling reaction is not completed. Afterwards, the Fmoc protecting group is removed to yield product of formula (II) (wherein $R_L$ represents the lateral chain of the amino acid used in the previously described coupling reaction) by treatment with an amine base solution such as a piperidine solution in DMF and/or a mixture of piperidine/DBU/toluene/DMF.

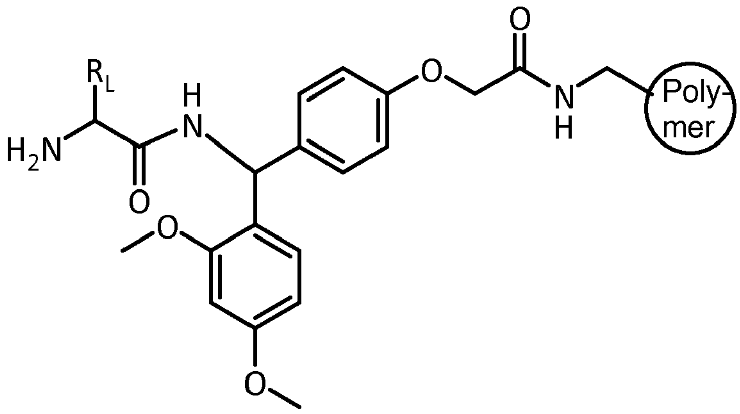

(II)

The subsequent amino acids of the peptide are coupled using the same procedure described above for the first amino acid.

Once all the amino acids have been coupled the resulting peptide (which is still bonded to the resin) is washed several times with DCM and dried by suction. Then, the peptide is cleaved from the resin using 95%TFA, 2.5% TIS and 2.5% water mixture. The acidic mixture and the resin containing the peptide are mixed and stirred at room temperature for 1-3 hours. Then, the reaction mixture is filtered and the polymeric support is rinsed with DCM. All the filtrates are pooled and the solvent is evaporated under a $N_2$ (flow stream) to yield the desired peptide.

## EXAMPLES

Example 1: Ac-Gly-D-Asp-D-Asn-D-Asp-D-Ser-D-Glu-D-Asp-D-Asn-D-Ser-D-Asp-D-Glu-D-lu-D-Asn-$NH_2$

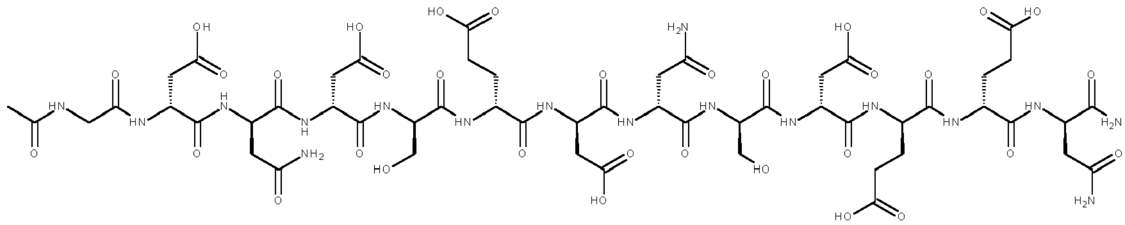

Polymeric support (resin): H-Rink Amide-ChemMatrix®
Used amino acids: Fmoc-D-Asn(Trt)-OH, Fmoc-D-Glu(O*t*Bu)-OH, Fmoc-D-Asp(O*t*Bu)-OH, Fmoc-D-Ser(*t*Bu)-OH and Fmoc-Gly-OH.

General procedure for synthesis:

Couplings: the corresponding amino acid (3 eq), Oxyma pure (3 eq) and DIC (3 eq) are dissolved in 2 mL of DMF. Two minutes later, the reaction mixture is added to the resin. For the coupling of the first amino acid to the resin, the reaction is allowed to proceed for 3h at room temperature with intermittent stirring. For the following amino acids the coupling time is 1 hour. Then, the reaction mixture is filtered off and the resin is washed with DMF (x5) and DCM (x5). Then, a colorimetric test is performed to check the completeness of the reaction. If the reaction is not complete, a recoupling in the same conditions is performed.

Fmoc removal is performed with treatments with a solution of 20% piperidine in DMF (2x5 min, 1 x 10 min). Washes with DMF (x5) and DCM (x5) are carried out after Fmoc removal. No colorimetric test is performed.

Acetylation: after last amino acid removal, the *N*-terminal part of the peptide is acetylated with 10 eq of DIEA and 10 eq of acetic anhydride with DCM during 15 minutes.

Cleavage: the resin is washed several times with DCM and dried. The peptide is cleaved from the resin by adding a mixture of 95% TFA, 2.5% TIS and 2.5% water at room temperature for 2 hours under intermittent stirring. The reaction mixture is filtered and rinsed with DCM (x5). All the filtrates are pooled and the solvent is evaporated using a $N_2$ stream. The crude of synthesis is resuspended in a mixture of water and acetonitrile (1:1), analyzed by HPLC and HPLC-MS. The crude of synthesis is purified by reverse phase chromatography, lyophilized and fully characterized.

Characterization of example 1 after purification:

| HPLC retention time (min)[1] | Calculated MW | Observed MW[2] | Purity (HPLC, Area/Area) |
|---|---|---|---|
| 1.55 | 1480.3 | 1502.4 $[M+Na]^+$ | > 99.9% |
| [1]HPLC: Column HPLC Sunfire C18 (4.6 mm x 100 mm, 3.5 μm); flux 1 mL/min; Gradient 0-100% B in 8 min (A= 0.045% TFA in $H_2O$ and B 0.036% TFA in ACN).<br>[2]UPLC-MS: Column C18 Acquity (2.1 mm x 50 mm, 1.7 μm, Waters); flux 0.6 mL/min; Gradient 0-100% B in 2 min (A= 0.1% formic acid in $H_2O$ and B 0.07% formic acid in ACN). | | | |

Example 2: Ac-D-Ser-D-Glu-D-Asp-D-Asn-D-Ser-D-Asp-D-Glu-D-Glu-D-Asn-$NH_2$

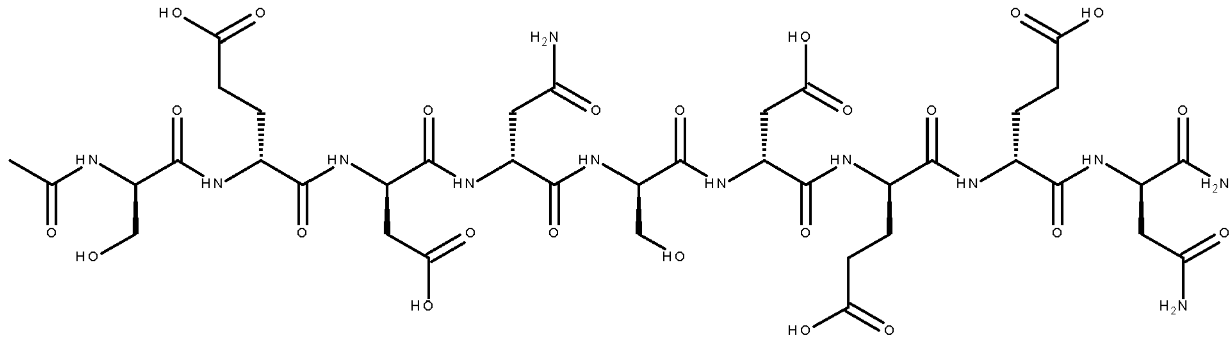

Polymeric support (resin): H-Rink Amide-ChemMatrix®
Used amino acids: Fmoc-D-Asn(Trt)-OH, Fmoc-D-Glu(O*t*Bu)-OH, Fmoc-D-Asp(O*t*Bu)-OH and Fmoc-D-Ser(*t*Bu)-OH.

General procedure for synthesis:

Couplings: the corresponding amino acid (3 eq), Oxyma pure (3 eq) and DIC (3 eq) are dissolved in 2 mL of DMF. Two minutes later, the reaction mixture is added to the resin. For the coupling of the first amino acid to the resin, the reaction is allowed to proceed for 3h at room temperature with intermittent stirring. For the following amino acids the coupling time is 1 hour. Then, the reaction mixture is filtered off and the resin is washed with DMF (x5) and DCM (x5). Then, a colorimetric test is performed to check the completeness of the reaction. If the reaction is not complete, a recoupling in the same conditions is performed.

Fmoc removal is performed with treatments with a solution of 20% piperidine in DMF (2x5 min, 1 x 10 min). Washes with DMF (x5) and DCM (x5) are carried out after Fmoc removal. No colorimetric test is performed.

Acetylation: after last amino acid removal, the *N*-terminal part of the peptide is acetylated with 10 eq of DIEA and 10 eq of acetic anhydride with DCM during 15 minutes.

Cleavage: the resin is washed several times with DCM and dried. The peptide is cleaved from the resin by adding a mixture of 95% TFA, 2.5% TIS and 2.5% water at room temperature for 2 hours under intermittent stirring. The reaction mixture is filtered and rinsed with DCM (x5). All the filtrates are pooled and the solvent is evaporated using a $N_2$ stream. The crude of synthesis is resuspended in a mixture of water and acetonitrile (1:1), analyzed by HPLC and HPLC-MS. The crude of synthesis is purified by reverse phase chromatography, lyophilized and fully characterized.

Characterization of example 2 after purification:

| HPLC retention time (min)[1] | Calculated MW | Observed MW[2] | Purity (HPLC, Area/Area) |
|---|---|---|---|
| 3.15 | 1078.9 | 1079.2 [M+H]$^+$ | > 90% |

[1]HPLC: Column HPLC Sunfire C18 (4.6 mm x 100 mm, 3.5 μm); flux 1 mL/min; Gradient 0-100% B in 8 min (A= 0.045% TFA in $H_2O$ and B 0.036% TFA in ACN).
[2]UPLC-MS: Column C18 Acquity (2.1 mm x 50 mm, 1.7 μm, Waters); flux 0.6 mL/min; Gradient 0-100% B in 2 min (A= 0.1% formic acid in $H_2O$ and B 0.07% formic acid in ACN).

Example 3: Ac-D-Ser-D-Glu-D-Asp-D-Asn-D-Ala-D-Asp-D-Glu-D-Glu-D-Ala-$NH_2$

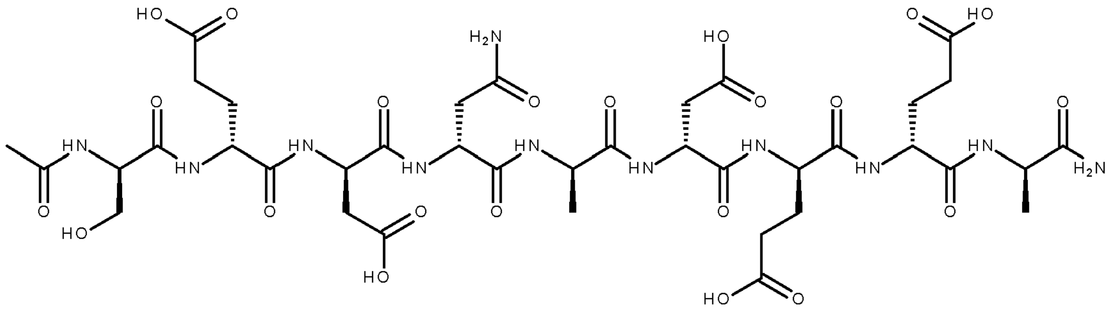

Polymeric support (resin): H-Rink Amide-ChemMatrix®
Used amino acids: Fmoc-D-Ala-OH, Fmoc-D-Asn(Trt)-OH, Fmoc-D-Glu(O*t*Bu)-OH, Fmoc-D-Asp(O*t*Bu)-OH and Fmoc-D-Ser(*t*Bu)-OH.

General procedure for synthesis:

Couplings: the corresponding amino acid (3 eq), Oxyma pure (3 eq) and DIC (3 eq) are dissolved in 2 mL of DMF. Two minutes later, the reaction mixture is added to the resin. For the coupling of the first amino acid to the resin, the reaction is allowed to proceed for 3h at room temperature with intermittent stirring. For the following amino acids the coupling time is 1 hour. Then, the reaction mixture is filtered off and the resin is washed with DMF (x5) and DCM (x5). Then, a colorimetric test is performed to check the completeness of the reaction. If the reaction is not complete, a recoupling in the same conditions is performed.

Fmoc removal is performed with treatments with a solution of 20% piperidine in DMF (2x5 min, 1 x 10 min). Washes with DMF (x5) and DCM (x5) are carried out after Fmoc removal. No colorimetric test is performed.

Acetylation: after last amino acid removal, the *N*-terminal part of the peptide is acetylated with 10 eq of DIEA and 10 eq of acetic anhydride with DCM during 15 minutes.

Cleavage: the resin is washed several times with DCM and dried. The peptide is cleaved from the resin by adding a mixture of 95% TFA, 2.5% TIS and 2.5% water at room temperature for 2 hours under intermittent stirring. The reaction mixture is filtered and rinsed with DCM (x5). All the filtrates are pooled and the solvent is evaporated using a $N_2$ stream. The crude of synthesis is resuspended in a mixture of water and acetonitrile (1:1), analyzed by HPLC and HPLC-MS. The crude of synthesis is purified by reverse phase chromatography, lyophilized and fully characterized.

Characterization of example 3 after purification:

| HPLC retention time (min)[1] | Calculated MW | Observed MW[2] | Purity (HPLC, Area/Area) |
|---|---|---|---|
| 3.34 | 1019.9 | 1020.3 [M+H]$^+$ | > 97% |

[1]HPLC: Column HPLC Sunfire C18 (4.6 mm x 100 mm, 3.5 μm); flux 1 mL/min; Gradient 0-100% B in 8 min (A= 0.045% TFA in $H_2O$ and B 0.036% TFA in ACN).
[2]UPLC-MS: Column C18 Acquity (2.1 mm x 50 mm, 1.7 μm, Waters); flux 0.6 mL/min; Gradient 0-100% B in 2 min (A= 0.1% formic acid in $H_2O$ and B 0.07% formic acid in ACN).

Example 4: Ac-D-Ser-D-Glu-D-Asp-D-Gln-D-Ser-D-Glu-D-Glu-D-Glu-D-Gln-$NH_2$

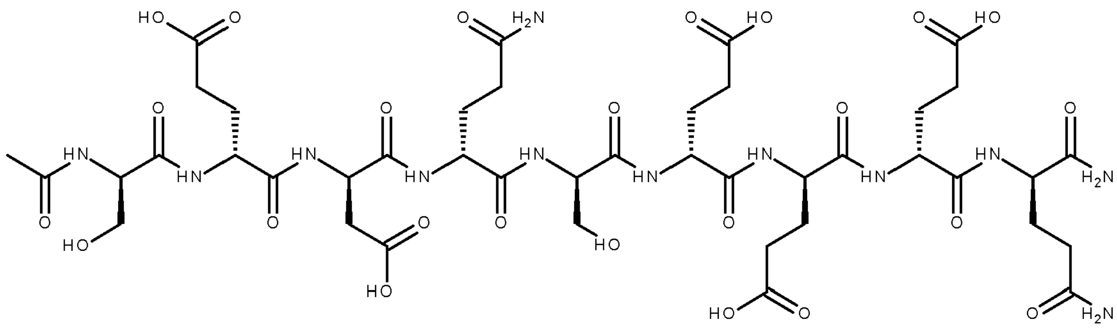

Polymeric support (resin): H-Rink Amide-ChemMatrix®
Used amino acids: Fmoc-D-Gln(Trt)-OH, Fmoc-D-Glu(O*t*Bu)-OH, Fmoc-D-Asp(O*t*Bu)-OH and Fmoc-D-Ser(*t*Bu)-OH.

General procedure for synthesis:

Couplings: the corresponding amino acid (3 eq), Oxyma pure (3 eq) and DIC (3 eq) are dissolved in 2 mL of DMF. Two minutes later, the reaction mixture is added to the resin. For the coupling of the first amino acid to the resin, the reaction is allowed to proceed for 3h at room temperature with intermittent stirring. For the following amino acids the coupling time is 1 hour. Then, the reaction mixture is filtered off and the resin is washed with DMF (x5) and DCM (x5). Then, a colorimetric test is performed to check the completeness of the reaction. If the reaction is not complete, a recoupling in the same conditions is performed.

Fmoc removal is performed with treatments with a solution of 20% piperidine in DMF (2x5 min, 1 x 10 min). Washes with DMF (x5) and DCM (x5) are carried out after Fmoc removal. No colorimetric test is performed.

Acetylation: after last amino acid removal, the *N*-terminal part of the peptide is acetylated with 10 eq of DIEA and 10 eq of acetic anhydride with DCM during 15 minutes.

Cleavage: the resin is washed several times with DCM and dried. The peptide is cleaved from the resin by adding a mixture of 95% TFA, 2.5% TIS and 2.5% water at room temperature for 2 hours under intermittent stirring. The reaction mixture is filtered and rinsed with DCM (x5). All the filtrates are pooled and the solvent is evaporated using a $N_2$ stream. The crude of synthesis is resuspended in a mixture of water and acetonitrile (1:1), analyzed by HPLC and HPLC-MS. The crude of synthesis is purified by reverse phase chromatography, lyophilized and fully characterized.

Characterization of example 4 after purification:

| HPLC retention time (min)[1] | Calculated MW | Observed MW[2] | Purity (HPLC, Area/Area) |
|---|---|---|---|
| 3.24 | 1121.0 | 1121.5 [M+H]$^+$ | > 91% |

[1]HPLC: Column HPLC Sunfire C18 (4.6 mm x 100 mm, 3.5 μm); flux 1 mL/min; Gradient 0-100% B in 8 min (A= 0.045% TFA in $H_2O$ and B 0.036% TFA in ACN).
[2]UPLC-MS: Column C18 Acquity (2.1 mm x 50 mm, 1.7 μm, Waters); flux 0.6 mL/min; Gradient 0-100% B in 2 min (A= 0.1% formic acid in $H_2O$ and B 0.07% formic acid in ACN).

Example 5: Ac-D-Ser-D-Glu-D-Asp-D-Gln-D-Ala-D-Glu-D-Glu-D-Glu-D-Ala-$NH_2$

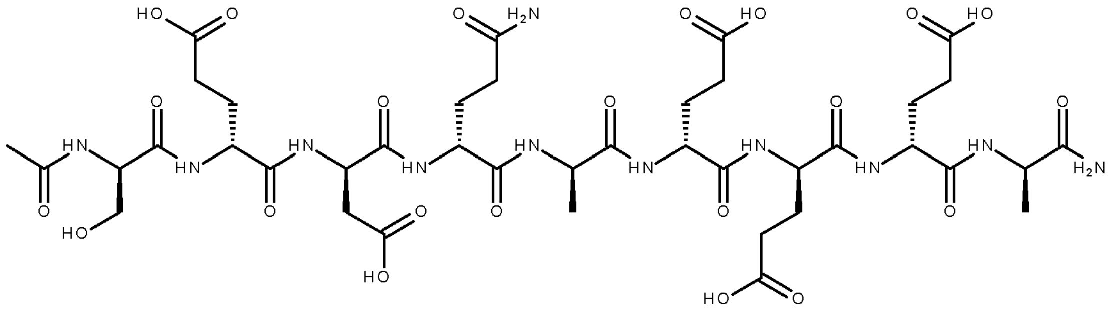

Polymeric support (resin): H-Rink Amide-ChemMatrix®
Used amino acids: Fmoc-D-Ala-OH. Fmoc-D-Gln(Trt)-OH, Fmoc-D-Glu(O*t*Bu)-OH, Fmoc-D-Asp(O*t*Bu)-OH and Fmoc-D-Ser(*t*Bu)-OH.

General procedure for synthesis:

Couplings: the corresponding amino acid (3 eq), Oxyma pure (3 eq) and DIC (3 eq) are dissolved in 2 mL of DMF. Two minutes later, the reaction mixture is added to the resin. For the coupling of the first amino acid to the resin, the reaction is allowed to proceed for 3h at room temperature with intermittent stirring. For the following amino acids the coupling time is 1 hour. Then, the reaction mixture is filtered off and the resin is washed with DMF (x5) and DCM (x5). Then, a colorimetric test is performed to check the completeness of the reaction. If the reaction is not complete, a recoupling in the same conditions is performed.

Fmoc removal is performed with treatments with a solution of 20% piperidine in DMF (2x5 min, 1 x 10 min). Washes with DMF (x5) and DCM (x5) are carried out after Fmoc removal. No colorimetric test is performed.

Acetylation: after last amino acid removal, the *N*-terminal part of the peptide is acetylated with 10 eq of DIEA and 10 eq of acetic anhydride with DCM during 15 minutes.

Cleavage: the resin is washed several times with DCM and dried. The peptide is cleaved from the resin by adding a mixture of 95% TFA, 2.5% TIS and 2.5% water at room temperature for 2 hours under intermittent stirring. The reaction mixture is filtered and rinsed with DCM (x5). All the filtrates are pooled and the solvent is evaporated using a $N_2$ stream. The crude of synthesis is resuspended in a mixture of water and acetonitrile (1:1), analyzed by HPLC and HPLC-MS. The crude of synthesis is purified by reverse phase chromatography, lyophilized and fully characterized.

Characterization of example 5 after purification:

| HPLC retention time (min)[1] | Calculated MW | Observed MW[2] | Purity (HPLC, Area/Area) |
|---|---|---|---|
| 3.47 | 1047.9 | 1048.2 $[M+H]^+$ | > 92% |

[1]HPLC: Column HPLC Sunfire C18 (4.6 mm x 100 mm, 3.5 μm); flux 1 mL/min; Gradient 0-100% B in 8 min (A= 0.045% TFA in $H_2O$ and B 0.036% TFA in ACN).
[2]UPLC-MS: Column C18 Acquity (2.1 mm x 50 mm, 1.7 μm, Waters); flux 0.6 mL/min; Gradient 0-100% B in 2 min (A= 0.1% formic acid in $H_2O$ and B 0.07% formic acid in ACN).

Example 6: Ac-D-Ser-D-Glu-D-Asp-D-Asn-D-Arg-D-Asp-D-Glu-D-Glu-D-Arg-$NH_2$

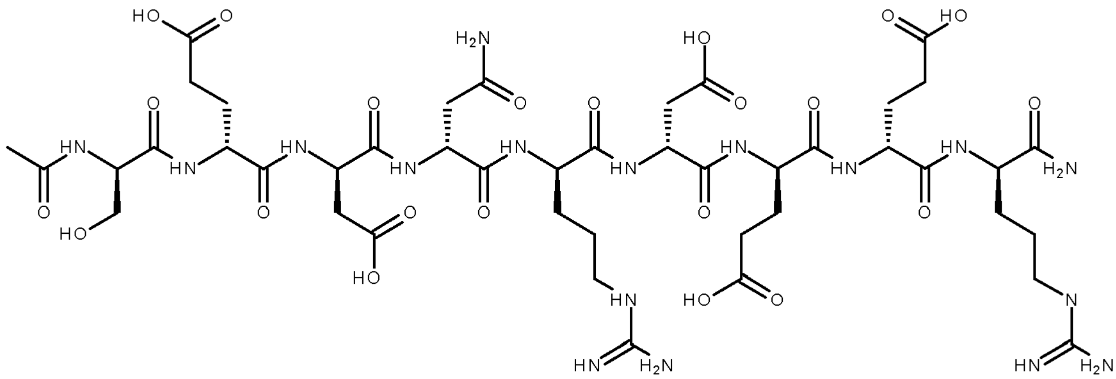

Polymeric support (resin): H-Rink Amide-ChemMatrix®
Used amino acids: Fmoc-D-Arg(pbf)-OH, Fmoc-D-Asn(Trt)-OH, Fmoc-D-Glu(O*t*Bu)-OH, Fmoc-D-Asp(O*t*Bu)-OH and Fmoc-D-Ser(*t*Bu)-OH.

General procedure for synthesis:

Couplings: the corresponding amino acid (3 eq), Oxyma pure (3 eq) and DIC (3 eq) are dissolved in 2 mL of DMF. Two minutes later, the reaction mixture is added to the resin. For the coupling of the first amino acid to the resin, the reaction is allowed to proceed for 3h at room temperature with intermittent stirring. For the following amino acids the coupling time is 1 hour. Then, the reaction mixture is filtered off and the resin is washed with DMF (x5) and DCM (x5). Then, a colorimetric test is performed to check the completeness of the reaction. If the reaction is not complete, a recoupling in the same conditions is performed.
Fmoc removal is performed with treatments with a solution of 20% piperidine in DMF (2x5 min, 1 x 10 min). Washes with DMF (x5) and DCM (x5) are carried out after Fmoc removal. No colorimetric test is performed.
Acetylation: after last amino acid removal, the *N*-terminal part of the peptide is acetylated with 10 eq of DIEA and 10 eq of acetic anhydride with DCM during 15 minutes.
Cleavage: the resin is washed several times with DCM and dried. The peptide is cleaved from the resin by adding a mixture of 95% TFA, 2.5% TIS and 2.5% water at room temperature for 2 hours under intermittent stirring. The reaction mixture is filtered and rinsed with DCM (x5). All the filtrates are pooled and the solvent is evaporated using a $N_2$ stream. The crude of synthesis is resuspended in a mixture of water and acetonitrile (1:1), analyzed by HPLC and HPLC-MS. The crude of synthesis is purified by reverse phase chromatography, lyophilized and fully characterized.

Characterization of example 6 after purification:

| HPLC retention time (min)[1] | Calculated MW | Observed MW[2] | Purity (HPLC, Area/Area) |
|---|---|---|---|
| 3.10 | 1190.1 | 1190.5 $[M+H]^+$ | > 94% |

[1]HPLC: Column HPLC Sunfire C18 (4.6 mm x 100 mm, 3.5 μm); flux 1 mL/min; Gradient 0-100% B in 8 min (A= 0.045% TFA in $H_2O$ and B 0.036% TFA in ACN).
[2]UPLC-MS: Column C18 Acquity (2.1 mm x 50 mm, 1.7 μm, Waters); flux 0.6 mL/min; Gradient 0-100% B in 2 min (A= 0.1% formic acid in $H_2O$ and B 0.07% formic acid in ACN).

Example 7: Ac-D-Ser-D-Glu-D-Asp-D-Asn-D-Arg-D-Asp-D-Glu-D-Glu-D-Leu-$NH_2$

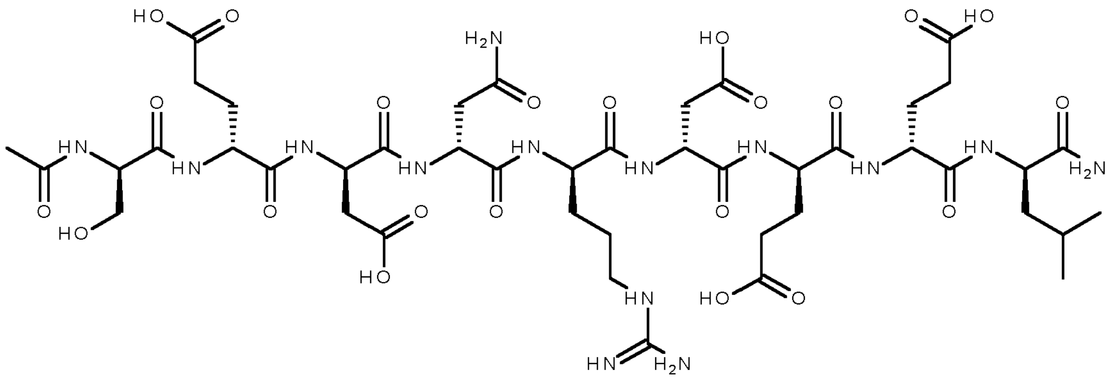

Polymeric support (resin): H-Rink Amide-ChemMatrix®
Used amino acids: Fmoc-D-Leu-OH, Fmoc-D-Arg(pbf)-OH, Fmoc-D-Asn(Trt)-OH, Fmoc-D-Glu(O*t*Bu)-OH, Fmoc-D-Asp(O*t*Bu)-OH, and Fmoc-D-Ser(*t*Bu)-OH.

General procedure for synthesis:

Couplings: the corresponding amino acid (3 eq), Oxyma pure (3 eq) and DIC (3 eq) are dissolved in 2 mL of DMF. Two minutes later, the reaction mixture is added to the resin. For the coupling of the first amino acid to the resin, the reaction is allowed to proceed for 3h at room temperature with intermittent stirring. For the following amino acids the coupling time is 1 hour. Then, the reaction mixture is filtered off and the resin is washed with DMF (x5) and DCM (x5). Then, a colorimetric test is performed to check the completeness of the reaction. If the reaction is not complete, a recoupling in the same conditions is performed.

Fmoc removal is performed with treatments with a solution of 20% piperidine in DMF (2x5 min, 1 x 10 min). Washes with DMF (x5) and DCM (x5) are carried out after Fmoc removal. No colorimetric test is performed.

Acetylation: after last amino acid removal, the *N*-terminal part of the peptide is acetylated with 10 eq of DIEA and 10 eq of acetic anhydride with DCM during 15 minutes.

Cleavage: the resin is washed several times with DCM and dried. The peptide is cleaved from the resin by adding a mixture of 95% TFA, 2.5% TIS and 2.5% water at room temperature for 2 hours under intermittent stirring. The reaction mixture is filtered and rinsed with DCM (x5). All the filtrates are pooled and the solvent is evaporated using a $N_2$ stream. The crude of synthesis is resuspended in a mixture of water and acetonitrile (1:1), analyzed by HPLC and HPLC-MS. The crude of synthesis is purified by reverse phase chromatography, lyophilized and fully characterized.

Characterization of example 7 after purification:

| HPLC retention time (min)[1] | Calculated MW | Observed MW[2] | Purity (HPLC, Area/Area) |
|---|---|---|---|
| 3.58 | 1147.1 | 1147.0 | > 98% |
| [1]HPLC: Column HPLC Sunfire C18 (4.6 mm x 100 mm, 3.5 μm); flux 1 mL/min; Gradient 0-100% B in 8 min (A= 0.045% TFA in $H_2O$ and B 0.036% TFA in ACN). [2]UPLC-MS: Column C18 Acquity (2.1 mm x 50 mm, 1.7 μm, Waters); flux 0.6 mL/min; Gradient 0-100% B in 2 min (A= 0.1% formic acid in $H_2O$ and B 0.07% formic acid in ACN). | | | |

Example 8: Ac-D-Ser-D-Glu-D-Asp-D-Asn-D-Arg-D-Asp-D-Glu-D-Glu-D-Trp-$NH_2$

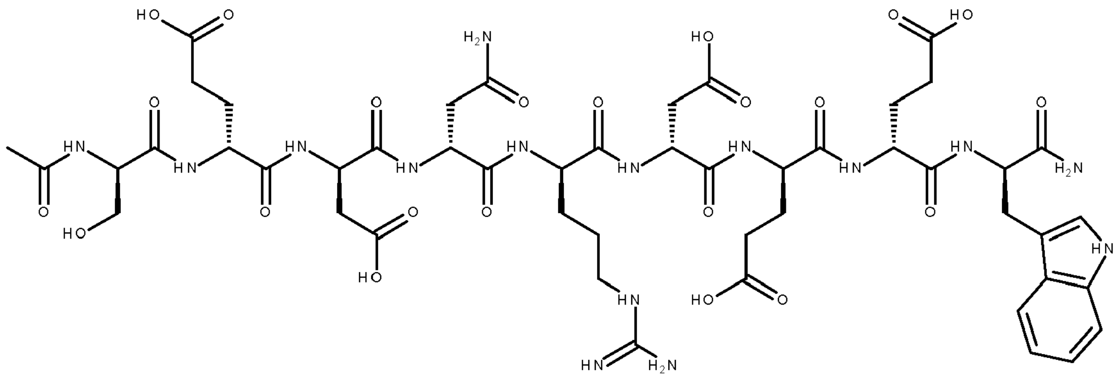

Polymeric support (resin): H-Rink Amide-ChemMatrix®
Used amino acids: Fmoc-D-Trp(Boc)-OH, Fmoc-D-Arg(pbf)-OH, Fmoc-D-Asn(Trt)-OH, Fmoc-D-Glu(O*t*Bu)-OH, Fmoc-D-Asp(O*t*Bu)-OH, and Fmoc-D-Ser(*t*Bu)-OH.

General procedure for synthesis:

Couplings: the corresponding amino acid (3 eq), Oxyma pure (3 eq) and DIC (3 eq) are dissolved in 2 mL of DMF. Two minutes later, the reaction mixture is added to the resin. For the coupling of the first amino acid to the resin, the reaction is allowed to proceed for 3h at room temperature with intermittent stirring. For the following amino acids the coupling time is 1 hour. Then, the reaction mixture is filtered off and the resin is washed with DMF (x5) and DCM (x5). Then, a colorimetric test is performed to check the completeness of the reaction. If the reaction is not complete, a recoupling in the same conditions is performed.
Fmoc removal is performed with treatments with a solution of 20% piperidine in DMF (2x5 min, 1 x 10 min). Washes with DMF (x5) and DCM (x5) are carried out after Fmoc removal. No colorimetric test is performed.
Acetylation: after last amino acid removal, the *N*-terminal part of the peptide is acetylated with 10 eq of DIEA and 10 eq of acetic anhydride with DCM during 15 minutes.
Cleavage: the resin is washed several times with DCM and dried. The peptide is cleaved from the resin by adding a mixture of 95% TFA, 2.5% TIS and 2.5% water at room temperature for 2 hours under intermittent stirring. The reaction mixture is filtered and rinsed with DCM (x5). All the filtrates are pooled and the solvent is evaporated using a $N_2$ stream. The crude of synthesis is resuspended in a mixture of water and acetonitrile (1:1), analyzed by HPLC and HPLC-MS. The crude of synthesis is purified by reverse phase chromatography, lyophilized and fully characterized.

Characterization of example 8 after purification:

| HPLC retention time (min)[1] | Calculated MW | Observed MW[2] | Purity (HPLC, Area/Area) |
|---|---|---|---|
| 3.93 | 1120.1 | 1120.4 $[M+H]^+$ | > 96% |
| [1]HPLC: Column HPLC Sunfire C18 (4.6 mm x 100 mm, 3.5 μm); flux 1 mL/min; Gradient 0-100% B in 8 min (A= 0.045% TFA in $H_2O$ and B 0.036% TFA in ACN).<br>[2]UPLC-MS: Column C18 Acquity (2.1 mm x 50 mm, 1.7 μm, Waters); flux 0.6 mL/min; Gradient 0-100% B in 2 min (A= 0.1% formic acid in $H_2O$ and B 0.07% formic acid in ACN). | | | |

Example 9: Ac-D-Ser-D-Glu-D-Asp-D-Asn-D-Lys-D-Asp-D-Glu-D-Glu-D-Asn-$NH_2$

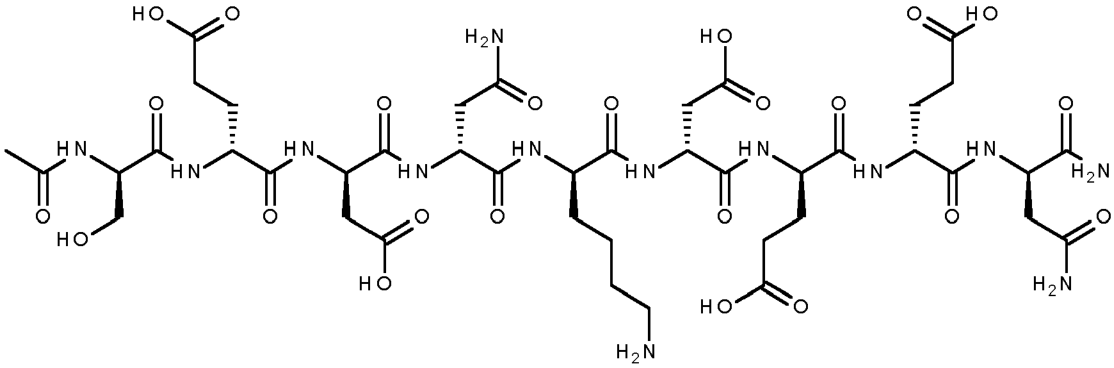

Polymeric support (resin): H-Rink Amide-ChemMatrix®
Used amino acids: Fmoc-D-Asn(Trt)-OH, Fmoc-D-Glu(O*t*Bu)-OH, Fmoc-D-Asp(O*t*Bu)-OH, Fmoc-D-Lys(Boc)-OH and Fmoc-D-Ser(*t*Bu)-OH.

General procedure for synthesis:

Couplings: the corresponding amino acid (3 eq), Oxyma pure (3 eq) and DIC (3 eq) are dissolved in 2 mL of DMF. Two minutes later, the reaction mixture is added to the resin. For the coupling of the first amino acid to the resin, the reaction is allowed to proceed for 3h at room temperature with intermittent stirring. For the following amino acids the coupling time is 1 hour. Then, the reaction mixture is filtered off and the resin is washed with DMF (x5) and DCM (x5). Then, a colorimetric test is performed to check the completeness of the reaction. If the reaction is not complete, a recoupling in the same conditions is performed.
Fmoc removal is performed with treatments with a solution of 20% piperidine in DMF (2x5 min, 1 x 10 min). Washes with DMF (x5) and DCM (x5) are carried out after Fmoc removal. No colorimetric test is performed.
Acetylation: after last amino acid removal, the *N*-terminal part of the peptide is acetylated with 10 eq of DIEA and 10 eq of acetic anhydride with DCM during 15 minutes.
Cleavage: the resin is washed several times with DCM and dried. The peptide is cleaved from the resin by adding a mixture of 95% TFA, 2.5% TIS and 2.5% water at room temperature for 2 hours under intermittent stirring. The reaction mixture is filtered and rinsed with DCM (x5). All the filtrates are pooled and the solvent is evaporated using a $N_2$ stream. The crude of synthesis is resuspended in a mixture of water and acetonitrile (1:1), analyzed by HPLC and HPLC-MS. The crude of synthesis is purified by reverse phase chromatography, lyophilized and fully characterized.

Characterization of example 9 after purification:

| HPLC retention time (min)[1] | Calculated MW | Observed MW[2] | Purity (HPLC, Area/Area) |
|---|---|---|---|
| 3.05 | 1120.0 | 1120.2 $[M+H]^+$ | > 93% |
| [1]HPLC: Column HPLC Sunfire C18 (4.6 mm x 100 mm, 3.5 μm); flux 1 mL/min; Gradient 0-100% B in 8 min (A= 0.045% TFA in $H_2O$ and B 0.036% TFA in ACN).<br>[2]UPLC-MS: Column C18 Acquity (2.1 mm x 50 mm, 1.7 μm, Waters); flux 0.6 mL/min; Gradient 0-100% B in 2 min (A= 0.1% formic acid in $H_2O$ and B 0.07% formic acid in ACN). | | | |

Example 10: Ac-D-Asp-D-Asn-D-Asp-D-Ser-D-Glu-D-Asp-D-Asn-D-Ser-D-Asp-D-Glu-D-Glu-D-Asn-$NH_2$

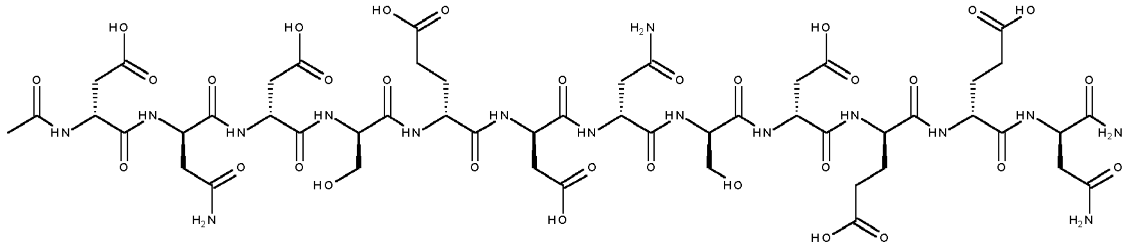

Polymeric support (resin): H-Rink Amide-ChemMatrix®
Used amino acids: Fmoc-D-Asn(Trt)-OH, Fmoc-D-Glu(O*t*Bu)-OH, Fmoc-D-Asp(O*t*Bu)-OH and Fmoc-D-Ser(*t*Bu)-OH.

General procedure for synthesis:

Couplings: the corresponding amino acid (3 eq), Oxyma pure (3 eq) and DIC (3 eq) are dissolved in 2 mL of DMF. Two minutes later, the reaction mixture is added to the resin. For the coupling of the first amino acid to the resin, the reaction is allowed to proceed for 3h at room temperature with intermittent stirring. For the following amino acids the coupling time is 1 hour. Then, the reaction mixture is filtered off and the resin is washed with DMF (x5) and DCM (x5). Then, a colorimetric test is performed to check the completeness of the reaction. If the reaction is not complete, a recoupling in the same conditions is performed.

Fmoc removal is performed with treatments with a solution of 20% piperidine in DMF (2x5 min, 1 x 10 min). Washes with DMF (x5) and DCM (x5) are carried out after Fmoc removal. No colorimetric test is performed.

Acetylation: after last amino acid removal, the *N*-terminal part of the peptide is acetylated with 10 eq of DIEA and 10 eq of acetic anhydride with DCM during 15 minutes.

Cleavage: the resin is washed several times with DCM and dried. The peptide is cleaved from the resin by adding a mixture of 95% TFA, 2.5% TIS and 2.5% water at room temperature for 2 hours under intermittent stirring. The reaction mixture is filtered and rinsed with DCM (x5). All the filtrates are pooled and the solvent is evaporated using a $N_2$ stream. The crude of synthesis is resuspended in a mixture of water and acetonitrile (1:1), analyzed by HPLC and HPLC-MS. The crude of synthesis is purified by reverse phase chromatography, lyophilized and fully characterized.

Characterization of example 10 after purification:

| HPLC retention time (min)[1] | Calculated MW | Observed MW[2] | Purity (HPLC, Area/Area) |
|---|---|---|---|
| 1.35 | 1423.2 | 1424.0 $[M+H]^+$ | > 99% |

[1]HPLC: Column HPLC Sunfire C18 (4.6 mm x 100 mm, 3.5 μm); flux 1 mL/min; Gradient 0-100% B in 8 min (A= 0.045% TFA in $H_2O$ and B 0.036% TFA in ACN).
[2]UPLC-MS: Column C18 Acquity (2.1 mm x 50 mm, 1.7 μm, Waters); flux 0.6 mL/min; Gradient 0-100% B in 2 min (A= 0.1% formic acid in $H_2O$ and B 0.07% formic acid in ACN).

Example 11: Ac-D-Asn-D-Asp-D-Ser-D-Glu-D-Asp-D-Asn-D-Ser-D-Asp-D-Glu-D-Glu-D-Asn-$NH_2$

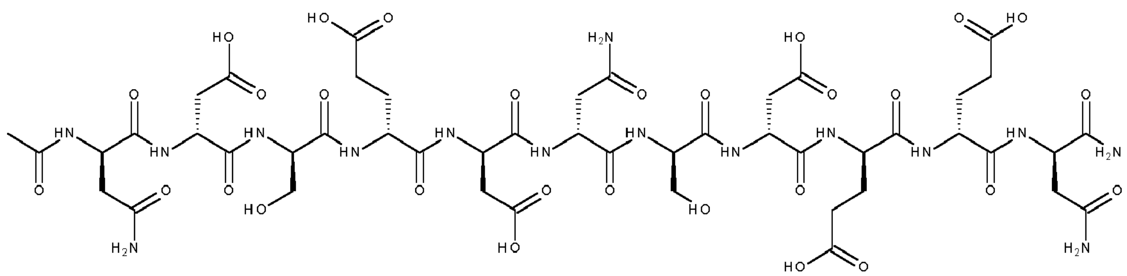

Polymeric support (resin): H-Rink Amide-ChemMatrix®

Used amino acids: Fmoc-D-Asn(Trt)-OH, Fmoc-D-Glu(O*t*Bu)-OH, Fmoc-D-Asp(O*t*Bu)-OH and Fmoc-D-Ser(*t*Bu)-OH.

General procedure for synthesis:

Couplings: the corresponding amino acid (3 eq), Oxyma pure (3 eq) and DIC (3 eq) are dissolved in 2 mL of DMF. Two minutes later, the reaction mixture is added to the resin. For the coupling of the first amino acid to the resin, the reaction is allowed to proceed for 3h at room temperature with intermittent stirring. For the following amino acids the coupling time is 1 hour. Then, the reaction mixture is filtered off and the resin is washed with DMF (x5) and DCM (x5). Then, a colorimetric test is performed to check the completeness of the reaction. If the reaction is not complete, a recoupling in the same conditions is performed.

Fmoc removal is performed with treatments with a solution of 20% piperidine in DMF (2x5 min, 1 x 10 min). Washes with DMF (x5) and DCM (x5) are carried out after Fmoc removal. No colorimetric test is performed.

Acetylation: after last amino acid removal, the *N*-terminal part of the peptide is acetylated with 10 eq of DIEA and 10 eq of acetic anhydride with DCM during 15 minutes.

Cleavage: the resin is washed several times with DCM and dried. The peptide is cleaved from the resin by adding a mixture of 95% TFA, 2.5% TIS and 2.5% water at room temperature for 2 hours under intermittent stirring. The reaction mixture is filtered and rinsed with DCM (x5). All the filtrates are pooled and the solvent is evaporated using a $N_2$ stream. The crude of synthesis is resuspended in a mixture of water and acetonitrile (1:1), analyzed by HPLC and HPLC-MS. The crude of synthesis is purified by reverse phase chromatography, lyophilized and fully characterized.

Characterization of example 11 after purification:

| HPLC retention time (min)[1] | Calculated MW | Observed MW[2] | Purity (HPLC, Area/Area) |
|---|---|---|---|
| 3.16 | 1308.1 | 1308.7 $[M+H]^+$ | > 91% |

[1]HPLC: Column HPLC Sunfire C18 (4.6 mm x 100 mm, 3.5 μm); flux 1 mL/min; Gradient 0-100% B in 8 min (A= 0.045% TFA in $H_2O$ and B 0.036% TFA in ACN).
[2]UPLC-MS: Column C18 Acquity (2.1 mm x 50 mm, 1.7 μm, Waters); flux 0.6 mL/min; Gradient 0-100% B in 2 min (A= 0.1% formic acid in $H_2O$ and B 0.07% formic acid in ACN).

Example 12: Ac-D-Asp-D-Ser-D-Glu-D-Asp-D-Asn-D-Ser-D-Asp-D-Glu-D-Glu-D-Asn-$NH_2$

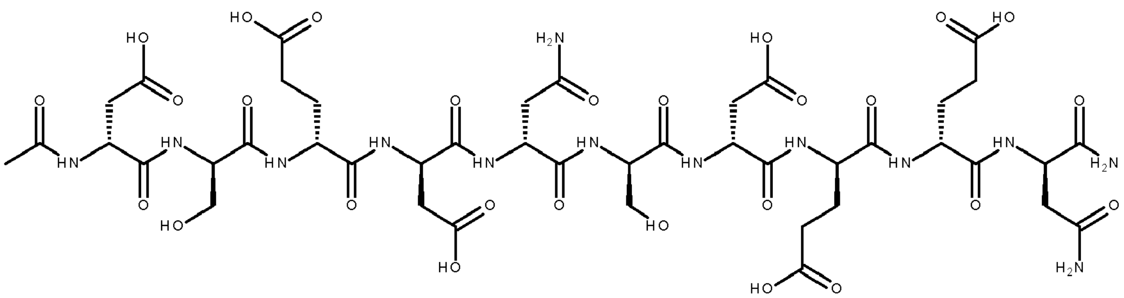

Polymeric support (resin): H-Rink Amide-ChemMatrix®
Used amino acids: Fmoc-D-Asn(Trt)-OH, Fmoc-D-Glu(O*t*Bu)-OH, Fmoc-D-Asp(O*t*Bu)-OH and Fmoc-D-Ser(*t*Bu)-OH.

General procedure for synthesis:

Couplings: the corresponding amino acid (3 eq), Oxyma pure (3 eq) and DIC (3 eq) are dissolved in 2 mL of DMF. Two minutes later, the reaction mixture is added to the resin. For the coupling of the first amino acid to the resin, the reaction is allowed to proceed for 3h at room temperature with intermittent stirring. For the following amino acids the coupling time is 1 hour. Then, the reaction mixture is filtered off and the resin is washed with DMF (x5) and DCM (x5). Then, a colorimetric test is performed to check the completeness of the reaction. If the reaction is not complete, a

recoupling in the same conditions is performed.

Fmoc removal is performed with treatments with a solution of 20% piperidine in DMF (2x5 min, 1 x 10 min). Washes with DMF (x5) and DCM (x5) are carried out after Fmoc removal. No colorimetric test is performed.

Acetylation: after last amino acid removal, the *N*-terminal part of the peptide is acetylated with 10 eq of DIEA and 10 eq of acetic anhydride with DCM during 15 minutes.

Cleavage: the resin is washed several times with DCM and dried. The peptide is cleaved from the resin by adding a mixture of 95% TFA, 2.5% TIS and 2.5% water at room temperature for 2 hours under intermittent stirring. The reaction mixture is filtered and rinsed with DCM (x5). All the filtrates are pooled and the solvent is evaporated using a $N_2$ stream. The crude of synthesis is resuspended in a mixture of water and acetonitrile (1:1), analyzed by HPLC and HPLC-MS. The crude of synthesis is purified by reverse phase chromatography, lyophilized and fully characterized.

Characterization of example 12 after purification:

| HPLC retention time (min)[1] | Calculated MW | Observed MW[2] | Purity (HPLC, Area/Area) |
|---|---|---|---|
| 3.17 | 1194.0 | 1194.2 | > 94% |

[1]HPLC: Column HPLC Sunfire C18 (4.6 mm x 100 mm, 3.5 μm); flux 1 mL/min; Gradient 0-100% B in 8 min (A= 0.045% TFA in $H_2O$ and B 0.036% TFA in ACN).
[2]UPLC-MS: Column C18 Acquity (2.1 mm x 50 mm, 1.7 μm, Waters); flux 0.6 mL/min; Gradient 0-100% B in 2 min (A= 0.1% formic acid in $H_2O$ and B 0.07% formic acid in ACN).

Example 13: Ac-D-Asp-D-Asn-D-Asp-D-Ser-D-Glu-D-Asp-D-Asn-D-Ser-D-Asp-D-Glu-D-Glu-D-Ala-$NH_2$

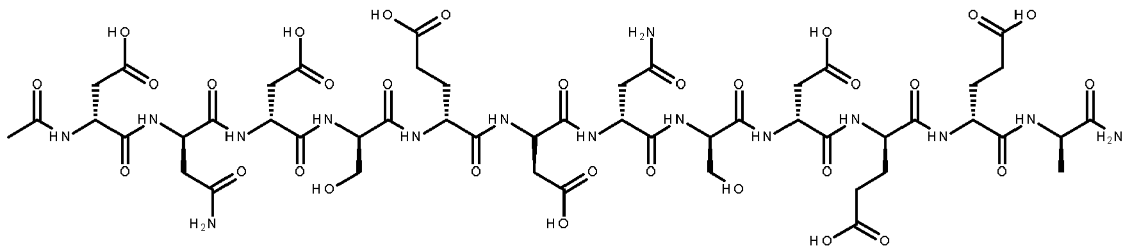

Polymeric support (resin): H-Rink Amide-ChemMatrix®
Used amino acids: Fmoc-D-Ala-OH, Fmoc-D-Asn(Trt)-OH, Fmoc-D-Glu(O*t*Bu)-OH, Fmoc-D-Asp(O*t*Bu)-OH and Fmoc-D-Ser(*t*Bu)-OH.

General procedure for synthesis:

Couplings: the corresponding amino acid (3 eq), Oxyma pure (3 eq) and DIC (3 eq) are dissolved in 2 mL of DMF. Two minutes later, the reaction mixture is added to the resin. For the coupling of the first amino acid to the resin, the reaction is allowed to proceed for 3h at room temperature with intermittent stirring. For the following amino acids the coupling time is 1 hour. Then, the reaction mixture is filtered off and the resin is washed with DMF (x5) and DCM (x5). Then, a colorimetric test is performed to check the completeness of the reaction. If the reaction is not complete, a recoupling in the same conditions is performed.

Fmoc removal is performed with treatments with a solution of 20% piperidine in DMF (2x5 min, 1 x 10 min). Washes with DMF (x5) and DCM (x5) are carried out after Fmoc removal. No colorimetric test is performed.

Acetylation: after last amino acid removal, the *N*-terminal part of the peptide is acetylated with 10 eq of DIEA and 10 eq of acetic anhydride with DCM during 15 minutes.

Cleavage: the resin is washed several times with DCM and dried. The peptide is cleaved from the resin by adding a mixture of 95% TFA, 2.5% TIS and 2.5% water at room temperature for 2 hours under intermittent stirring. The reaction mixture is filtered and rinsed with DCM (x5). All the filtrates are pooled and the solvent is evaporated using a $N_2$ stream. The crude of synthesis is resuspended in a mixture of water and acetonitrile (1:1), analyzed by HPLC and HPLC-MS. The crude of synthesis is purified by reverse phase chromatography, lyophilized and fully characterized.

Characterization of example 13 after purification:

| HPLC retention time (min)[1] | Calculated MW | Observed MW[2] | Purity (HPLC, Area/Area) |
|---|---|---|---|
| 1.40 | 1380.2 | 1381.1 [M+H]+ | > 99% |
| [1]HPLC: Column C18 XSelect CSH (50 mm x 4.6 mm, 3.5 μm, 100 Å, Waters); Flow rate 1.6 mL/min; Gradient 0-100% in 3.5 min (A=0.1% TFA in $H_2O$ and B 0.1% TFA in ACN).<br>[2]HPLC-MS: Column C18 XSelect CSH (50 mm x 4.6 mm, 3.5 μm, 100 Å, Waters); Flow rate 1.6 mL/min T=50°C; Gradient= 5-100% B in 3.5 min (A= 0.1% formic acid in $H_2O$, and B= 0.1% formic acid in ACN). | | | |

Example 14: Ac-D-Asn-D-Asp-D-Ser-D-Glu-D-Asp-D-Asn-D-Ser-D-Asp-D-Glu-D-Glu-D-Ala-$NH_2$

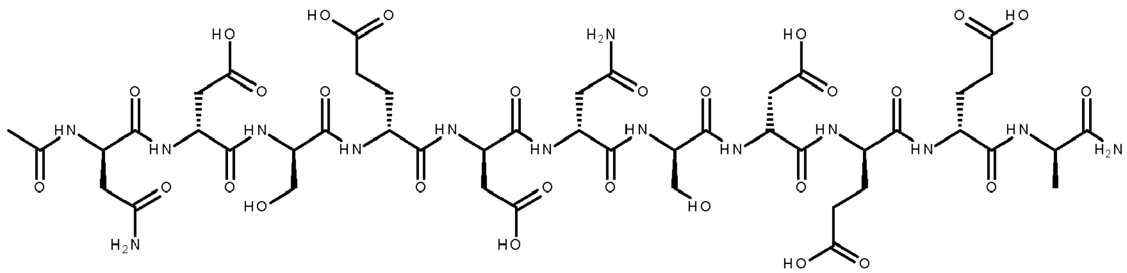

Polymeric support (resin): H-Rink Amide-ChemMatrix®
Used amino acids: Fmoc-D-Ala-OH, Fmoc-D-Asn(Trt)-OH, Fmoc-D-Glu(O*t*Bu)-OH, Fmoc-D-Asp(O*t*Bu)-OH and Fmoc-D-Ser(*t*Bu)-OH.

General procedure for synthesis:

Couplings: the corresponding amino acid (3 eq), Oxyma pure (3 eq) and DIC (3 eq) are dissolved in 2 mL of DMF. Two minutes later, the reaction mixture is added to the resin. For the coupling of the first amino acid to the resin, the reaction is allowed to proceed for 3h at room temperature with intermittent stirring. For the following amino acids the coupling time is 1 hour. Then, the reaction mixture is filtered off and the resin is washed with DMF (x5) and DCM (x5). Then, a colorimetric test is performed to check the completeness of the reaction. If the reaction is not complete, a recoupling in the same conditions is performed.
Fmoc removal is performed with treatments with a solution of 20% piperidine in DMF (2x5 min, 1 x 10 min). Washes with DMF (x5) and DCM (x5) are carried out after Fmoc removal. No colorimetric test is performed.
Acetylation: after last amino acid removal, the *N*-terminal part of the peptide is acetylated with 10 eq of DIEA and 10 eq of acetic anhydride with DCM during 15 minutes.
Cleavage: the resin is washed several times with DCM and dried. The peptide is cleaved from the resin by adding a mixture of 95% TFA, 2.5% TIS and 2.5% water at room temperature for 2 hours under intermittent stirring. The reaction mixture is filtered and rinsed with DCM (x5). All the filtrates are pooled and the solvent is evaporated using a $N_2$ stream. The crude of synthesis is resuspended in a mixture of water and acetonitrile (1:1), analyzed by HPLC and HPLC-MS. The crude of synthesis is purified by reverse phase chromatography, lyophilized and fully characterized.

Characterization of example 14 after purification:

| HPLC retention time (min)[1] | Calculated MW | Observed MW[2] | Purity (HPLC, Area/Area) |
|---|---|---|---|

(continued)

| HPLC retention time (min)[1] | Calculated MW | Observed MW[2] | Purity (HPLC, Area/Area) |
|---|---|---|---|
| 3.24 | 1265.0 | 1265.3 | > 95% |
| [1]HPLC: Column HPLC Sunfire C18 (4.6 mm x 100 mm, 3.5 μm); flux 1 mL/min; Gradient 0-100% B in 8 min (A= 0.045% TFA in $H_2O$ and B 0.036% TFA in ACN).<br>[2]UPLC-MS: Column C18 Acquity (2.1 mm x 50 mm, 1.7 μm, Waters); flux 0.6 mL/min; Gradient 0-100% B in 2 min (A= 0.1% formic acid in $H_2O$ and B 0.07% formic acid in ACN). | | | |

Example 15: Ac-D-Asp-D-Ser-D-Glu-D-Asp-D-Asn-D-Ser-D-Asp-D-Glu-D-Glu-D-Ala-$NH_2$

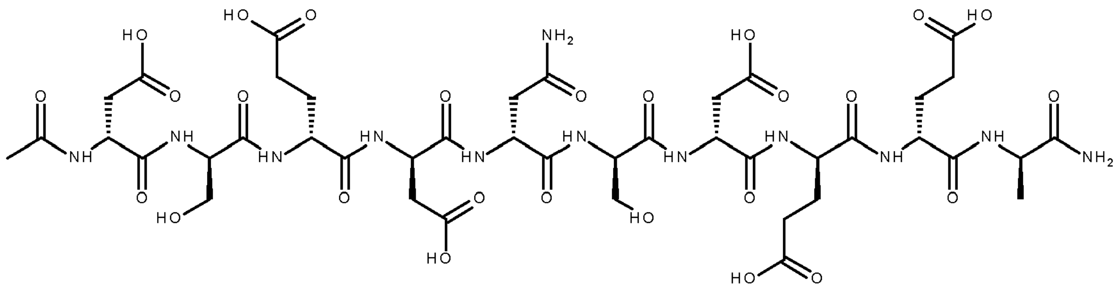

Polymeric support (resin): H-Rink Amide-ChemMatrix®
Used amino acids: Fmoc-D-Ala-OH, Fmoc-D-Asn(Trt)-OH, Fmoc-D-Glu(O*t*Bu)-OH, Fmoc-D-Asp(O*t*Bu)-OH and Fmoc-D-Ser(*t*Bu)-OH.

General procedure for synthesis:

Couplings: the corresponding amino acid (3 eq), Oxyma pure (3 eq) and DIC (3 eq) are dissolved in 2 mL of DMF. Two minutes later, the reaction mixture is added to the resin. For the coupling of the first amino acid to the resin, the reaction is allowed to proceed for 3h at room temperature with intermittent stirring. For the following amino acids the coupling time is 1 hour. Then, the reaction mixture is filtered off and the resin is washed with DMF (x5) and DCM (x5). Then, a colorimetric test is performed to check the completeness of the reaction. If the reaction is not complete, a recoupling in the same conditions is performed.

Fmoc removal is performed with treatments with a solution of 20% piperidine in DMF (2x5 min, 1 x 10 min). Washes with DMF (x5) and DCM (x5) are carried out after Fmoc removal. No colorimetric test is performed.

Acetylation: after last amino acid removal, the *N*-terminal part of the peptide is acetylated with 10 eq of DIEA and 10 eq of acetic anhydride with DCM during 15 minutes.

Cleavage: the resin is washed several times with DCM and dried. The peptide is cleaved from the resin by adding a mixture of 95% TFA, 2.5% TIS and 2.5% water at room temperature for 2 hours under intermittent stirring. The reaction mixture is filtered and rinsed with DCM (x5). All the filtrates are pooled and the solvent is evaporated using a $N_2$ stream. The crude of synthesis is resuspended in a mixture of water and acetonitrile (1:1), analyzed by HPLC and HPLC-MS. The crude of synthesis is purified by reverse phase chromatography, lyophilized and fully characterized.

Characterization of example 15 after purification:

| HPLC retention time (min)[1] | Calculated MW | Observed MW[2] | Purity (HPLC, Area/Area) |
|---|---|---|---|

(continued)

| HPLC retention time (min)[1] | Calculated MW | Observed MW[2] | Purity (HPLC, Area/Area) |
|---|---|---|---|
| 3.28 | 1151.0 | 1172.9 [M+Na]$^+$ | > 97% |

[1]HPLC: Column HPLC Sunfire C18 (4.6 mm x 100 mm, 3.5 μm); flux 1 mL/min; Gradient 0-100% B in 8 min (A= 0.045% TFA in $H_2O$ and B 0.036% TFA in ACN).
[2]UPLC-MS: Column C18 Acquity (2.1 mm x 50 mm, 1.7 μm, Waters); flux 0.6 mL/min; Gradient 0-100% B in 2 min (A= 0.1% formic acid in $H_2O$ and B 0.07% formic acid in ACN).

Example 16: Ac-D-Asp-D-Ser-D-Glu-D-Asp-D-Asn-D-Ala-D-Asp-D-Glu-D-Glu-D-Ala-$NH_2$

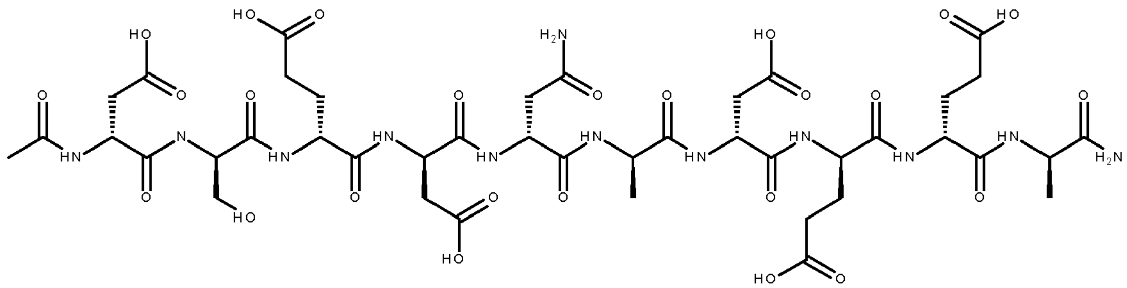

Polymeric support (resin): H-Rink Amide-ChemMatrix®
Used amino acids: Fmoc-D-Ala-OH, Fmoc-D-Asn(Trt)-OH, Fmoc-D-Glu(O*t*Bu)-OH, Fmoc-D-Asp(O*t*Bu)-OH and Fmoc-D-Ser(*t*Bu)-OH.

General procedure for synthesis:

Couplings: the corresponding amino acid (3 eq), Oxyma pure (3 eq) and DIC (3 eq) are dissolved in 2 mL of DMF. Two minutes later, the reaction mixture is added to the resin. For the coupling of the first amino acid to the resin, the reaction is allowed to proceed for 3h at room temperature with intermittent stirring. For the following amino acids the coupling time is 1 hour. Then, the reaction mixture is filtered off and the resin is washed with DMF (x5) and DCM (x5). Then, a colorimetric test is performed to check the completeness of the reaction. If the reaction is not complete, a recoupling in the same conditions is performed.

Fmoc removal is performed with treatments with a solution of 20% piperidine in DMF (2x5 min, 1 x 10 min). Washes with DMF (x5) and DCM (x5) are carried out after Fmoc removal. No colorimetric test is performed.

Acetylation: after last amino acid removal, the *N*-terminal part of the peptide is acetylated with 10 eq of DIEA and 10 eq of acetic anhydride with DCM during 15 minutes.

Cleavage: the resin is washed several times with DCM and dried. The peptide is cleaved from the resin by adding a mixture of 95% TFA, 2.5% TIS and 2.5% water at room temperature for 2 hours under intermittent stirring. The reaction mixture is filtered and rinsed with DCM (x5). All the filtrates are pooled and the solvent is evaporated using a $N_2$ stream. The crude of synthesis is resuspended in a mixture of water and acetonitrile (1:1), analyzed by HPLC and HPLC-MS. The crude of synthesis is purified by reverse phase chromatography, lyophilized and fully characterized.

Characterization of example 16 after purification:

| HPLC retention time (min)[1] | Calculated MW | Observed MW[2] | Purity (HPLC, Area/Area) |
|---|---|---|---|

(continued)

| HPLC retention time (min)[1] | Calculated MW | Observed MW[2] | Purity (HPLC, Area/Area) |
|---|---|---|---|
| 1.34 | 1135.0 | 1135.6 $[M+H]^+$<br>568.6 $[M+H]^{2+}$ | > 90% |

[1]HPLC: Column C18 XSelect CSH (50 mm x 4.6 mm, 3.5 μm, 100 Å, Waters); Flow rate 1.6 mL/min; Gradient 0-100% in 3.5 min (A=0.1% TFA in $H_2O$ and B 0.1% TFA in ACN).
[2]HPLC-MS: Column C18 XSelect CSH (50 mm x 4.6 mm, 3.5 μm, 100 Å, Waters); Flow rate 1.6 mL/min T=50°C; Gradient= 5-100% B in 3.5 min (A= 0.1% formic acid in $H_2O$, and B= 0.1% formic acid in ACN).

Example 17: Ac-D-Asp-D-Ala-D-Glu-D-Asp-D-Asn-D-Ala-D-Asp-D-Glu-D-Glu-D-Ala-NH2

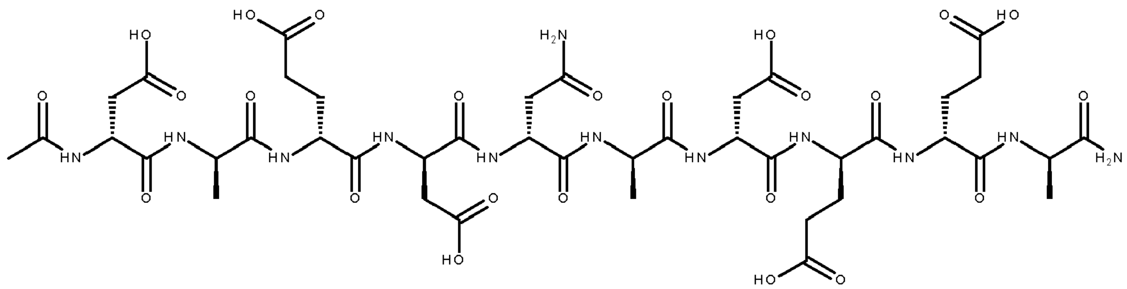

Polymeric support (resin): H-Rink Amide-ChemMatrix®
Used amino acids: Fmoc-D-Ala-OH, Fmoc-D-Asn(Trt)-OH, Fmoc-D-Glu(O*t*Bu)-OH and Fmoc-D-Asp(O*t*Bu)-OH.

General procedure for synthesis:

Couplings: the corresponding amino acid (3 eq), Oxyma pure (3 eq) and DIC (3 eq) are dissolved in 2 mL of DMF. Two minutes later, the reaction mixture is added to the resin. For the coupling of the first amino acid to the resin, the reaction is allowed to proceed for 3h at room temperature with intermittent stirring. For the following amino acids the coupling time is 1 hour. Then, the reaction mixture is filtered off and the resin is washed with DMF (x5) and DCM (x5). Then, a colorimetric test is performed to check the completeness of the reaction. If the reaction is not complete, a recoupling in the same conditions is performed.

Fmoc removal is performed with treatments with a solution of 20% piperidine in DMF (2x5 min, 1 x 10 min). Washes with DMF (x5) and DCM (x5) are carried out after Fmoc removal. No colorimetric test is performed.

Acetylation: after last amino acid removal, the *N*-terminal part of the peptide is acetylated with 10 eq of DIEA and 10 eq of acetic anhydride with DCM during 15 minutes.

Cleavage: the resin is washed several times with DCM and dried. The peptide is cleaved from the resin by adding a mixture of 95% TFA, 2.5% TIS and 2.5% water at room temperature for 2 hours under intermittent stirring. The reaction mixture is filtered and rinsed with DCM (x5). All the filtrates are pooled and the solvent is evaporated using a $N_2$ stream. The crude of synthesis is resuspended in a mixture of water and acetonitrile (1:1), analyzed by HPLC and HPLC-MS. The crude of synthesis is purified by reverse phase chromatography, lyophilized and fully characterized.

Characterization of example 17 after purification:

| HPLC retention time (min)[1] | Calculated MW | Observed MW[2] | Purity (HPLC, Area/Area) |
|---|---|---|---|

(continued)

| HPLC retention time (min)[1] | Calculated MW | Observed MW[2] | Purity (HPLC, Area/Area) |
|---|---|---|---|
| 1.45 | 1119.0 | 1119.8 $[M+H]^+$ | > 94% |

[1]HPLC: Column C18 XSelect CSH (50 mm x 4.6 mm, 3.5 μm, 100 Å, Waters); Flow rate 1.6 mL/min; Gradient 0-100% in 3.5 min (A=0.1% TFA in $H_2O$ and B 0.1% TFA in ACN).
[2]HPLC-MS: Column C18 XSelect CSH (50 mm x 4.6 mm, 3.5 μm, 100 Å, Waters); Flow rate 1.6 mL/min T=50°C; Gradient= 5-100% B in 3.5 min (A= 0.1% formic acid in $H_2O$, and B= 0.1% formic acid in ACN).

Example 18: Ac-D-Asp-D-Ser-D-Glu-D-Asp-D-Asn-D-Lys-D-Asp-D-Glu-D-Glu-D-Asn-$NH_2$

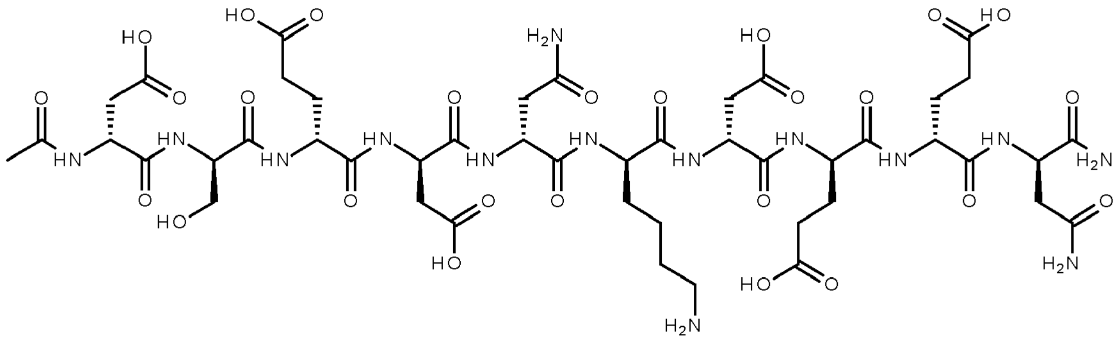

Polymeric support (resin): H-Rink Amide-ChemMatrix®
Used amino acids: Fmoc-D-Asn(Trt)-OH, Fmoc-D-Glu(O*t*Bu)-OH, Fmoc-D-Asp(O*t*Bu)-OH, Fmoc-D-Lys(Boc)-OH and Fmoc-D-Ser(*t*Bu)-OH.

General procedure for synthesis:

Couplings: the corresponding amino acid (3 eq), Oxyma pure (3 eq) and DIC (3 eq) are dissolved in 2 mL of DMF. Two minutes later, the reaction mixture is added to the resin. For the coupling of the first amino acid to the resin, the reaction is allowed to proceed for 3h at room temperature with intermittent stirring. For the following amino acids the coupling time is 1 hour. Then, the reaction mixture is filtered off and the resin is washed with DMF (x5) and DCM (x5). Then, a colorimetric test is performed to check the completeness of the reaction. If the reaction is not complete, a recoupling in the same conditions is performed.
Fmoc removal is performed with treatments with a solution of 20% piperidine in DMF (2x5 min, 1 x 10 min). Washes with DMF (x5) and DCM (x5) are carried out after Fmoc removal. No colorimetric test is performed.
Acetylation: after last amino acid removal, the *N*-terminal part of the peptide is acetylated with 10 eq of DIEA and 10 eq of acetic anhydride with DCM during 15 minutes.
Cleavage: the resin is washed several times with DCM and dried. The peptide is cleaved from the resin by adding a mixture of 95% TFA, 2.5% TIS and 2.5% water at room temperature for 2 hours under intermittent stirring. The reaction mixture is filtered and rinsed with DCM (x5). All the filtrates are pooled and the solvent is evaporated using a $N_2$ stream. The crude of synthesis is resuspended in a mixture of water and acetonitrile (1:1), analyzed by HPLC and HPLC-MS. The crude of synthesis is purified by reverse phase chromatography, lyophilized and fully characterized.

Characterization of example 18 after purification:

| HPLC retention time (min)[1] | Calculated MW | Observed MW[2] | Purity (HPLC, Area/Area) |
|---|---|---|---|

(continued)

| HPLC retention time (min)[1] | Calculated MW | Observed MW[2] | Purity (HPLC, Area/Area) |
|---|---|---|---|
| 1.32 | 1235.1 | 1236.0 $[M+H]^+$ | > 95% |

[1]HPLC: Column C18 XSelect CSH (50 mm x 4.6 mm, 3.5 μm, 100 Å, Waters); Flow rate 1.6 mL/min; Gradient 0-100% in 3.5 min (A=0.1% TFA in $H_2O$ and B 0.1% TFA in ACN).
[2]HPLC-MS: Column C18 XSelect CSH (50 mm x 4.6 mm, 3.5 μm, 100 Å, Waters); Flow rate 1.6 mL/min T=50°C; Gradient= 5-100% B in 3.5 min (A= 0.1% formic acid in $H_2O$, and B= 0.1% formic acid in ACN).

Example 19: Ac-D-Asp-D-Ser-D-Glu-D-Asp-D-Asn-D-Lys-D-Asp-D-Glu-D-Glu-D-Ala-$NH_2$

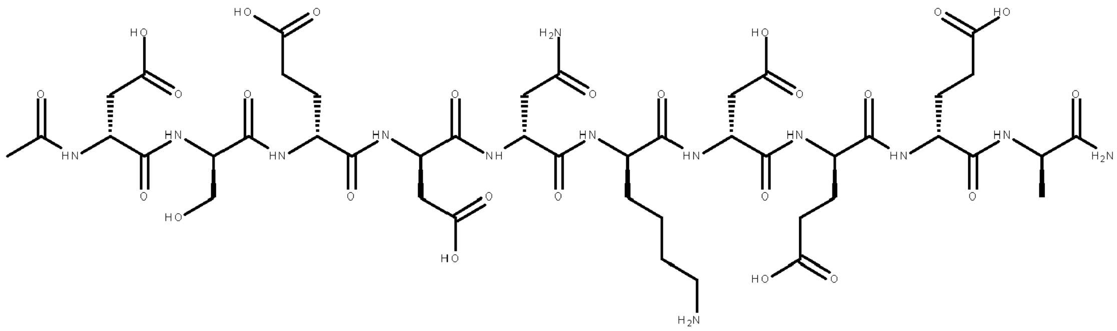

Polymeric support (resin): H-Rink Amide-ChemMatrix®
Used amino acids: Fmoc-D-Ala-OH, Fmoc-D-Asn(Trt)-OH, Fmoc-D-Glu(O*t*Bu)-OH, Fmoc-D-Asp(O*t*Bu)-OH, Fmoc-D-Lys(Boc)-OH and Fmoc-D-Ser(*t*Bu)-OH.

General procedure for synthesis:

Couplings: the corresponding amino acid (3 eq), Oxyma pure (3 eq) and DIC (3 eq) are dissolved in 2 mL of DMF. Two minutes later, the reaction mixture is added to the resin. For the coupling of the first amino acid to the resin, the reaction is allowed to proceed for 3h at room temperature with intermittent stirring. For the following amino acids the coupling time is 1 hour. Then, the reaction mixture is filtered off and the resin is washed with DMF (x5) and DCM (x5). Then, a colorimetric test is performed to check the completeness of the reaction. If the reaction is not complete, a recoupling in the same conditions is performed.

Fmoc removal is performed with treatments with a solution of 20% piperidine in DMF (2x5 min, 1 x 10 min). Washes with DMF (x5) and DCM (x5) are carried out after Fmoc removal. No colorimetric test is performed.

Acetylation: after last amino acid removal, the *N*-terminal part of the peptide is acetylated with 10 eq of DIEA and 10 eq of acetic anhydride with DCM during 15 minutes.

Cleavage: the resin is washed several times with DCM and dried. The peptide is cleaved from the resin by adding a mixture of 95% TFA, 2.5% TIS and 2.5% water at room temperature for 2 hours under intermittent stirring. The reaction mixture is filtered and rinsed with DCM (x5). All the filtrates are pooled and the solvent is evaporated using a $N_2$ stream. The crude of synthesis is resuspended in a mixture of water and acetonitrile (1:1), analyzed by HPLC and HPLC-MS. The crude of synthesis is purified by reverse phase chromatography, lyophilized and fully characterized.

Characterization of example 19 after purification:

| HPLC retention time (min)[1] | Calculated MW | Observed MW[2] | Purity (HPLC, Area/Area) |
|---|---|---|---|

(continued)

| HPLC retention time (min)[1] | Calculated MW | Observed MW[2] | Purity (HPLC, Area/Area) |
|---|---|---|---|
| 1.42 | 1192.1 | 1193.0 $[M+H]^+$ | > 92% |
| [1]HPLC: Column C18 XSelect CSH (50 mm x 4.6 mm, 3.5 μm, 100 Å, Waters); Flow rate 1.6 mL/min; Gradient 0-100% in 3.5 min (A=0.1% TFA in $H_2O$ and B 0.1% TFA in ACN).<br>[2]HPLC-MS: Column C18 XSelect CSH (50 mm x 4.6 mm, 3.5 μm, 100 Å, Waters); Flow rate 1.6 mL/min T=50°C; Gradient= 5-100% B in 3.5 min (A= 0.1% formic acid in $H_2O$, and B= 0.1% formic acid in ACN). | | | |

## Experiments:

### Determination of inhibitory effect of novel compounds on human LDL aggregation

The efficacy of the novel compounds to inhibit the aggregation of LDL has been tested in two parallel turbidimetric assays differing from each other only in the enzyme used to induce LDL aggregation, namely sphingomyelinase (SMase) and phospholipase $A_2$ (PLA2), respectively.

### LDL isolation and purification

Human LDL ($d_{1.019}$-$d_{1.063}$ g/mL) were obtained from pooled normolipemic human plasma by sequential ultracentrifugation in a KBr density gradient. Briefly, VLDL were first discarded after spinning plasma at 36.000 rpm for 18 h at 4°C, VLDL-free plasma was layered with 1.063 g/mL KBr solution and centrifuged at 36,000 rpm for 18 h at 4°C. LDL were dialyzed against 0.02 M Tris, 0.15 M NaCl, 1 mM EDTA, pH 7.5 for 18 hours, and then against normal saline for 2 hours. Finally, isolated LDL were filter-sterilized. Protein concentration was determined using a colorimetric assay.

### Anti-LDL aggregation assay

Human LDL particles (1.44 mg/mL) were incubated with 40 U/L of *Bacillus cereus* SMase or with 50 μg/L of type II secretory PLA2 from honey bee venom in 20 mM Tris buffer (pH 7.0) containing 150 mM NaCl, 2 mM $CaCl_2$, and $MgCl_2$ at 37°C. LDL incubation with SMase and PLA2 was performed at peptide concentration of 10 μM (ratio compound/ApoB-100: 4.7/1) for 18 hours. LDL lipolysis was stopped by addition of EDTA (final concentration 10 mM).
The efficiency of each novel compound to inhibit LDL aggregation induced by SMase and PLA2 was estimated by turbidimetry measuring the absorbance at a wavelength of 405 nm. Thus, the inhibitory activity was calculated according to Equation I.

$$\text{Inhibition activity} = [1-(a-b/c-b)]*100$$

(Equation I)

wherein:

- a corresponds to the absorbance value in the presence of nLDL particles, LDL aggregating enzyme (i.e., SMase or PLA2) and test compound;
- b corresponds to the absorbance value in the presence of only nLDL particles;
- c corresponds to the absorbance value in the presence of nLDL particles and LDL aggregating enzyme (i.e., SMase or PLA2);

The inhibitory activity of each novel compound on LDL aggregation induced by either SMase and PLA2 is detailed in Table 1.

**Table 1:** Inhibition of LDL aggregation

| Example | SMase-treated LDL | PLA2-treated LDL |
|---|---|---|
| 1 | 92.5 | 77.5 |

(continued)

| Example | SMase-treated LDL | PLA2-treated LDL |
|---|---|---|
| 2 | 80.7 | 87.7 |
| 3 | 90.0 | 37.2 |
| 4 | 91.5 | 32.6 |
| 5 | 90.1 | 20.7 |
| 6 | 85.0 | 15.3 |
| 7 | 44.7 | -1.9 |
| 8 | 64.2 | 3.1 |
| 9 | 91.0 | 36.4 |
| 10 | 96.0 | 94.0 |
| 11 | 94.8 | 95.2 |
| 12 | 93.3 | 94.3 |
| 13 | 95.5 | 95.9 |
| 14 | 68.3 | 74.0 |
| 15 | 96.0 | 95.9 |
| 16 | 94.0 | 61.9 |
| 17 | 95.2 | 66.1 |
| 18 | 85.1 | 55.1 |
| 19 | 87.5 | 46.2 |

**Determination of inhibitory effect of novel compounds on the intracellular cholesterol accumulation induced by SMase**

Human coronary vascular smooth muscle cells (hVSMC) exposed to LDL (nLDL or SMase-LDL) showed similar free cholesterol (FC) levels than hVSMC unexposed to LDL, indicating that LDL did not alter FC content in these cells. Conversely, intracellular cholesteryl esters (CE) detected in these cells upon exposure to LDL derives exclusively from CE supplied by LDL as hcVSMC unexposed to LDL did not have intracellular cholesteryl esters (CE).

Thus, the inhibitory effect of the novel compounds on the intracellular cholesterol accumulation has been analysed in terms of decrease in the ratio of intracellular CE and free cholesterol FC content of hcVSMC exposed to LDL and SMase-LDL.

**Culture of human coronary vascular smooth cells (hVSMC)**

All cells used were from a unique batch in order to prevent variability from cell origin. Cell quiescence was induced by maintaining the cell culture for 24 hours in a medium. Serum-deprived cells between passages 4 and 8 were used for experiments. VSMCs at these passages appeared as a relatively homogeneous population, showing a hill-and-valley confluence pattern. Cell monolayers were grown in vascular cell basal medium supplemented with vascular smooth muscle growth kit components. Quiescent cells were 2-hours exposed to LDL treated with SMase (in absence or presence of peptides) overnight at a concentration of 10 $\mu$M previously checked its aggregation by a turbidimetry test. Cells were then either collected for lipid extraction or processed for confocal microscopy analysis.

**Lipid extraction and determination of free and cholesteryl ester content**

Following the lipoprotein incubation period, hcVSMC were exhaustively washed (twice with PBS, twice with PBS supplemented with 1% BSA, and once with PBS supplemented with both 1% BSA and 100 U/mL heparin), before they were harvested into 1 mL of 0.15 M NaOH. Lipids were extracted using the Bligh and Dyer method. The lipid extract was redissolved in dichloromethane, applied to silica gel plates, and separated by thin layer chromatography. A mixture

of cholesterol and cholesterol palmitate, were also run as standards. Heptane or a solvent combination of heptane/diethylether/acetic acid (74:21:4, v/v/v) were used as chromatographic mobile phase. After lipid separation, the plates were dried and stained as previously reported. Finally, the spots corresponding to cholesteryl ester (CE) and free cholesterol (FC) were quantitated by densitometry.

The efficacy of each novel compound to decrease the ratio of intracellular cholesteryl esters (CE) and free cholesterol (FC) content of hcVSMC exposed to LDL and SMase-LDL was calculated according to Equation II.

$$\text{Efficacy} = [1\text{-}(a\text{-}b/c\text{-}b)]*100$$

(Equation II)

wherein:

a corresponds to the CE/FC ratio in hcVSMC exposed to SMase-LDL and in the presence of the test compound.
b corresponds to the CE/FC ratio in hcVSMC exposed to nLDL in the absence of the test compound.
c corresponds to the CE/FC ratio in hcVSMC exposed to SMase-LDL in the absence of the test compound.

The inhibitory effect of each novel compound on the intracellular cholesterol accumulation induced by SMase is detailed in Table 2.

**Table 2:** inhibitory effect of each novel compound on the intracellular cholesterol accumulation induced by SMase.

| Efficacy is measured as the decrease in the ratio between intracellular cholesteryl esters (CE) and free cholesterol (FC) content of human coronary vascular smooth muscle cells (hcVSMC) exposed to LDL and SMase-LDL | |
|---|---|
| **Example** | **CE/FC ratio decrease (%)** |
| 1 | 76.5 |
| 2 | 58.8 |
| 3 | N/A |
| 4 | N/A |
| 5 | N/A |
| 6 | 58.8 |
| 7 | N/A |
| 8 | N/A |
| 9 | 58.8 |
| 10 | 78.4 |
| 11 | 76.5 |
| 12 | 76.5 |
| 13 | 80.4 |
| 14 | 62.7 |
| 15 | 90.2 |
| 16 | 69.5 |
| 17 | 67.4 |
| 18 | 47.6 |
| 19 | 17.7 |

The following are particular embodiments of the present invention:

Embodiment 1: A compound of formula (I):

$$R^1\text{-}AA^1\text{-}AA^2\text{-}AA^3\text{-}AA^4\text{-}AA^5\text{-D-Glu-D-Asp-}AA^6\text{-}AA^7\text{-}AA^8\text{-D-Glu-D-Glu-}AA^9\text{-}NH_2 \quad (I)$$

wherein

$R^1$ is a $C_{2-4}$ acyl group, preferably acetyl,
$AA^1$ is either absent or is Gly
$AA^2$ is either absent or is D-Asp
$AA^3$ is either absent or is D-Asn
$AA^4$ is either absent or is D-Asp
$AA^5$ is selected from the group consisting of D-Ser and D-Ala
$AA^6$ is selected from the group consisting of D-Asn and D-Gln
$AA^7$ is selected from the group consisting of D-Ala, D-Arg, D-Lys and D-Ser
$AA^8$ is selected from the group consisting of D-Asp and D-Glu
$AA^9$ is selected from the group consisting of D-Ala, D-Arg, D-Asn, D-Gln, D-Leu and D-Trp

or a salt or solvate thereof.

Embodiment 2: A compound according to Embodiment 1 wherein $R^1$ is acetyl.

Embodiment 3: A compound according to anyone of Embodiments 1 to 2 wherein $AA^1$ is absent.

Embodiment 4: A compound according to anyone of Embodiments 1 to 3 wherein $AA^2$ is absent.

Embodiment 5: A compound according to anyone of Embodiments 1 to 4 wherein $AA^3$ is absent.

Embodiment 6: A compound according to Embodiment 1 wherein -$AA^1$-$AA^2$-$AA^3$-$AA^4$-$AA^5$- is either absent or is selected from the group consisting of -D-Gly-D-Asp-D-Asn-D-Asp-D-Ser, -D-Asp-D-Asn-D-Asp-D-Ser- and -D-Asn-D-Asp-D-Ser-, -D-Asp-D-Ser and -D-Asp-D-Ala-.

Embodiment 7: A compound according to anyone of Embodiments 1 to 6 wherein $AA^5$ is D-Ser.

Embodiment 8: A compound according to anyone of Embodiments 1 to 7 wherein - $AA^6$-$AA^7$-$AA^8$- is selected from the group consisting of -D-Asn-D-Ser-D-Asp-, -D-Asn-D-Ala-D-Asp-,.-D-Gln-D-Ser-D-Glu-, -D-Gln-D-Ala-D-Glu-, -D-Asn-D-Arg-D-Asp- and -D-Asn-D-Lys-D-Asp-.

Embodiment 9: A compound according to Embodiment 8 wherein -$AA^6$-$AA^7$-$AA^8$- is -D-Asn-D-Ser-D-Asp-.

Embodiment 10: A compound according to anyone of Embodiments 1 to 9 wherein -$AA^9$- is selected from the group consisting of D-Ala and D-Asn.

Embodiment 11: A compound according to Embodiment 1 selected from the group consisting of:

- Ac-Gly-D-Asp-D-Asn-D-Asp-D-Ser-D-Glu-D-Asp-D-Asn-D-Ser-D-Asp-D-Glu-D-Glu-D-Asn-$NH_2$
- Ac-D-Ser-D-Glu-D-Asp-D-Asn-D-Ser-D-Asp-D-Glu-D-Glu-D-Asn-$NH_2$
- Ac-D-Ser-D-Glu-D-Asp-D-Asn-D-Ala-D-Asp-D-Glu-D-Glu-D-Ala-$NH_2$
- Ac-D-Ser-D-Glu-D-Asp-D-Gln-D-Ser-D-Glu-D-Glu-D-Glu-D-Gln-$NH_2$
- Ac-D-Ser-D-Glu-D-Asp-D-Gln-D-Ala-D-Glu-D-Gglu-D-Glu-D-Ala-$NH_2$
- Ac-D-Ser-D-Glu-D-Asp-D-Asn-D-Arg-D-Asp-D-Glu-D-Glu-D-Arg-$NH_2$
- Ac-D-Ser-D-Glu-D-Asp-D-Asn-D-Arg-D-Asp-D-Glu-D-Glu-D-Leu-$NH_2$
- Ac-D-Ser-D-Glu-D-Asp-D-Asn-D-Arg-D-Asp-D-Glu-D-Glu-D-Trp-$NH_2$
- Ac-D-Ser-D-Glu-D-Asp-D-Asn-D-Lys-D-Asp-D-Glu-D-Glu-D-Asn-$NH_2$
- Ac-D-Asp-D-Asn-D-Asp-D-Ser-D-Glu-D-Asp-D-Asn-D-Ser-D-Asp-D-Glu-D-Glu-D-Asn-$NH_2$
- Ac-D-Asn-D-Asp-D-Ser-D-Glu-D-Asp-D-Asn-D-Ser-D-Asp-D-Glu-D-Glu-D-Asn-$NH_2$
- Ac-D-Asp-D-Ser-D-Glu-D-Asp-D-Asn-D-Ser-D-Asp-D-Glu-D-Glu-D-Asn-$NH_2$
- Ac-D-Asp-D-Asn-D-Asp-D-Ser-D-Glu-D-Asp-D-Asn-D-Ser-D-Asp-D-Glu-D-Glu-D-Ala-$NH_2$
- Ac-D-Asn-D-Asp-D-Ser-D-Glu-D-Asp-D-Asn-D-Ser-D-Asp-D-Glu-D-Glu-D-Ala-$NH_2$

- Ac-D-Asp-D-Ser-D-Glu-D-Asp-D-Asn-D-Ser-D-Asp-D-Glu-D-Glu-D-Ala-$NH_2$
- Ac-D-Asp-D-Ser-D-Glu-D-Asp-D-Asn-D-Ala-D-Asp-D-Glu-D-Glu-D-Ala-$NH_2$
- Ac-D-Asp-D-Ala-D-Glu-D-Asp-D-Asn-D-Ala-D-Asp-D-Glu-D-Glu-D-Ala-$NH_2$
- Ac-D-Asp-D-Ser-D-Glu-D-Asp-D-Asn-D-Lys-D-Asp-D-Glu-D-Glu-D-Asn-$NH_2$
- Ac-D-Asp-D-Ser-D-Glu-D-Asp-D-Asn-D-Lys-D-Asp-D-Glu-D-Glu-D-Ala-$NH_2$

or a pharmaceutically acceptable salt or solvate thereof.

Embodiment 12: Pharmaceutical compositions comprising a compound according to anyone of Embodiments 1 to 11 and a pharmaceutically acceptable carrier, adjuvant or vehicle.

Embodiment 13: A compound according to anyone of Embodiments 1 to 11 for use as a medicament.

Embodiment 14: A compound according to anyone of Embodiments 1 to 11 for use in the prevention and/or treatment of conditions wherein decrease of vascular cholesterol accumulation, inhibition of LDL aggregation and/or prevention of aggregated LDL (agLDL) internalization is useful.

Embodiment 15: A compound for use according to Embodiment 14 wherein the condition is selected from the group consisting of atherosclerosis, coronary artery disease, stroke, peripheral artery disease, angina pectoris, thrombosis, hyperlipidemia, hyperlipoproteinemia type II, familial hypercholesterolemia, familial combined hyperlipidemia, type II diabetes, hypothyroidism, Cushing’s syndrome and obesity.

## Claims

**1.** A compound of formula (I):

$$R^1\text{-}AA^1\text{-}AA^2\text{-}AA^3\text{-}AA^4\text{-}AA^5\text{-D-Glu-D-Asp-}AA^6\text{-}AA^7\text{-}AA^8\text{-D-Glu-D-Glu-}AA^9\text{-}NH_2 \quad \text{(I)}$$

wherein

$R^1$ is a $C_{2-4}$ acyl group,
$AA^1$ is either absent or is Gly
$AA^2$ is either absent or is D-Asp

$AA^3$ is either absent or is D-Asn
$AA^4$ is either absent or is D-Asp
$AA^5$ is selected from the group consisting of D-Ser and D-Ala
$AA^6$ is selected from the group consisting of D-Asn and D-Gln
$AA^7$ is selected from the group consisting of D-Ala, D-Arg, D-Lys and D-Ser
$AA^8$ is selected from the group consisting of D-Asp and D-Glu
$AA^9$ is selected from the group consisting of D-Ala, D-Arg, D-Asn, D-Gln, D-Leu and D-Trp

or a salt or solvate thereof.

**2.** A compound according to claim 1 wherein $R^1$ is acetyl.

**3.** A compound according to anyone of claims 1 to 2 wherein $AA^1$ is absent.

**4.** A compound according to anyone of claims 1 to 3 wherein $AA^2$ is absent.

**5.** A compound according to anyone of claims 1 to 4 wherein $AA^3$ is absent.

**6.** A compound according to claim 1 wherein $-AA^1-AA^2-AA^3-AA^4-AA^5-$ is either absent or is selected from the group consisting of -Gly-D-Asp-D-Asn-D-Asp-D-Ser, - D-Asp-D-Asn-D-Asp-D-Ser- and -D-Asn-D-Asp-D-Ser-, -D-Asp-D-Ser and -D-Asp-D-Ala-.

**7.** A compound according to anyone of claims 1 to 6 wherein $AA^5$ is D-Ser.

**8.** A compound according to anyone of claims 1 to 7 wherein $-AA^6-AA^7-AA^8-$ is selected from the group consisting of -D-Asn-D-Ser-D-Asp-, -D-Asn-D-Ala-D-Asp-,-D-Gln-D-Ser-D-Glu-, -D-Gln-D-Ala-D-Glu-, -D-Asn-D-Arg-D-Asp- and -D-Asn-D-Lys-D-Asp-.

**9.** A compound according to claim 8 wherein $-AA^6-AA^7-AA^8-$ is -D-Asn-D-Ser-D-Asp-.

**10.** A compound according to anyone of claims 1 to 9 wherein $-AA^9-$ is selected from the group consisting of D-Ala and D-Asn.

**11.** A compound according to claim 1 selected from the group consisting of:

- Ac-Gly-D-Asp-D-Asn-D-Asp-D-Ser-D-Glu-D-Asp-D-Asn-D-Ser-D-Asp-D-Glu-D-Glu-D-Asn-$NH_2$
- Ac-D-Ser-D-Glu-D-Asp-D-Asn-D-Ser-D-Asp-D-Glu-D-Glu-D-Asn-$NH_2$
- Ac-D-Ser-D-Glu-D-Asp-D-Asn-D-Ala-D-Asp-D-Glu-D-Glu-D-Ala-$NH_2$
- Ac-D-Ser-D-Glu-D-Asp-D-Gln-D-Ser-D-Glu-D-Glu-D-Glu-D-Gln-$NH_2$
- Ac-D-Ser-D-Glu-D-Asp-D-Gln-D-Ala-D-Glu-D-Gglu-D-Glu-D-Ala-$NH_2$
- Ac-D-Ser-D-Glu-D-Asp-D-Asn-D-Arg-D-Asp-D-Glu-D-Glu-D-Arg-$NH_2$
- Ac-D-Ser-D-Glu-D-Asp-D-Asn-D-Arg-D-Asp-D-Glu-D-Glu-D-Leu-$NH_2$
- Ac-D-Ser-D-Glu-D-Asp-D-Asn-D-Arg-D-Asp-D-Glu-D-Glu-D-Trp-$NH_2$
- Ac-D-Ser-D-Glu-D-Asp-D-Asn-D-Lys-D-Asp-D-Glu-D-Glu-D-Asn-$NH_2$
- Ac-D-Asp-D-Asn-D-Asp-D-Ser-D-Glu-D-Asp-D-Asn-D-Ser-D-Asp-D-Glu-D-Glu-D-Asn-$NH_2$
- Ac-D-Asn-D-Asp-D-Ser-D-Glu-D-Asp-D-Asn-D-Ser-D-Asp-D-Glu-D-Glu-D-Asn-$NH_2$
- Ac-D-Asp-D-Ser-D-Glu-D-Asp-D-Asn-D-Ser-D-Asp-D-Glu-D-Glu-D-Asn-$NH_2$
- Ac-D-Asp-D-Asn-D-Asp-D-Ser-D-Glu-D-Asp-D-Asn-D-Ser-D-Asp-D-Glu-D-Glu-D-Ala-$NH_2$
- Ac-D-Asn-D-Asp-D-Ser-D-Glu-D-Asp-D-Asn-D-Ser-D-Asp-D-Glu-D-Glu-D-Ala-$NH_2$
- Ac-D-Asp-D-Ser-D-Glu-D-Asp-D-Asn-D-Ser-D-Asp-D-Glu-D-Glu-D-Ala-$NH_2$
- Ac-D-Asp-D-Ser-D-Glu-D-Asp-D-Asn-D-Ala-D-Asp-D-Glu-D-Glu-D-Ala-$NH_2$
- Ac-D-Asp-D-Ala-D-Glu-D-Asp-D-Asn-D-Ala-D-Asp-D-Glu-D-Glu-D-Ala-$NH_2$
- Ac-D-Asp-D-Ser-D-Glu-D-Asp-D-Asn-D-Lys-D-Asp-D-Glu-D-Glu-D-Asn-$NH_2$
- Ac-D-Asp-D-Ser-D-Glu-D-Asp-D-Asn-D-Lys-D-Asp-D-Glu-D-Glu-D-Ala-$NH_2$

or a pharmaceutically acceptable salt or solvate thereof.

**12.** Pharmaceutical compositions comprising a compound according to anyone of claims 1 to 11 and a pharmaceutically

acceptable carrier, adjuvant or vehicle.

**13.** A compound according to anyone of claims 1 to 11 for use as a medicament.

**14.** A compound according to anyone of claims 1 to 11 for use in the prevention and/or treatment of conditions wherein decrease of vascular cholesterol accumulation, inhibition of LDL aggregation; prevention of aggregated LDL (agLDL) internalization and/or inhibition or reduction of the formation of VSMC foam cells is useful.

**15.** A compound for use according to claim 14 wherein the condition is selected from the group consisting of atherosclerosis, coronary artery disease, stroke, peripheral artery disease, angina pectoris, thrombosis, hyperlipidemia, hyperlipoproteinemia type II, familial hypercholesterolemia, familial combined hyperlipidemia, type II diabetes, hypothyroidism, Cushing's syndrome and obesity.

Europäisches Patentamt
European Patent Office
Office européen des brevets

# EUROPEAN SEARCH REPORT

Application Number
EP 19 38 2335

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PAULA COSTALES ET AL: "K Domain CR9 of Low Density Lipoprotein (LDL) Receptor-related Protein 1 (LRP1) Is Critical for Aggregated LDL-induced Foam Cell Formation from Human Vascular Smooth Muscle Cells", JOURNAL OF BIOLOGICAL CHEMISTRY, vol. 290, no. 24, 27 April 2015 (2015-04-27), pages 14852-14865, XP055628700, US ISSN: 0021-9258, DOI: 10.1074/jbc.M115.638361 * abstract; figures 11,12 * ----- | 1-15 | INV. C07K14/705 A61K38/00 |
| A | NAVAB M ET AL: "Oral administration of an Apo A-I mimetic peptide synthesized from D-amino acids dramatically reduces atherosclerosis in mice independent of plasma cholesterol", CIRCULATION, AMERICAN HEART ASSOCIATION, US, vol. 105, no. 3, 22 January 2002 (2002-01-22), pages 290-292, XP002378224, ISSN: 0009-7322, DOI: 10.1161/HC0302.103711 * abstract; figure 1 * * page 290 * ----- -/-- | 1-15 | TECHNICAL FIELDS SEARCHED (IPC): C07K A61K |

The present search report has been drawn up for all claims

| Place of search | Date of completion of the search | Examiner |
|---|---|---|
| The Hague | 3 October 2019 | Schmidt-Yodlee, H |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 19 38 2335

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | ALEYDA BENITEZ-AMARO ET AL: "Molecular basis for the protective effects of low-density lipoprotein receptor-related protein 1 (LRP1)-derived peptides against LDL aggregation", BIOCHIMICA ET BIOPHYSICA ACTA (BBA) - BIOMEMBRANES, vol. 1861, no. 7, 8 May 2019 (2019-05-08), pages 1302-1316, XP055628684, AMSTERDAM, NL ISSN: 0005-2736, DOI: 10.1016/j.bbamem.2019.05.003 * the whole document * ----- | 1-15 | |
| | | | TECHNICAL FIELDS SEARCHED (IPC) |

The present search report has been drawn up for all claims

1

| Place of search | Date of completion of the search | Examiner |
|---|---|---|
| The Hague | 3 October 2019 | Schmidt-Yodlee, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BLOOM, D.E. ; CAFIERO, E.T. ; JANÉ-LLOPIS, E. ; ABRAHAMS-GESSEL, S. ; BLOOM, L.R. ; FATHIMA, S. ; FEIGL, A.B. ; GAZIANO, T. ; MOWAFI, M. ; PANDYA, A.** The Global Economic Burden of Non-communicable Diseases. World Economic Forum, 2011
- **YUSUF S et al.** *Lancet,* 11 September 2004, vol. 364 (9438), 937-52
- **CASTELLI WP et al.** *Ann Epidemiol.,* January 1992, vol. 2 (1-2), 23-8
- **GORDON T et al.** *Am J Med.,* May 1977, vol. 62 (5), 707-14
- **BJÖRCK L. et al.** *Eur Heart J.,* May 2009, vol. 30 (9), 1046-56
- **BANDOSZ P. et al.** *BMJ,* 25 January 2012, vol. 344, d8136
- **WIJEYSUNDERA HC et al.** *JAMA,* 12 May 2010, vol. 303 (18), 1841-7
- **FLORES-MATEO G.** *Rev Esp Cardiol.,* November 2011, vol. 64 (11), 988-96
- **HUGHES J. et al.** *Eur J Prev Cardiol.,* April 2013, vol. 20 (2), 310-21
- **FORD ES et al.** *N Engl J Med.,* 07 June 2007, vol. 356 (23), 2388-98
- **ASPELUND T. et al.** *PLoS One,* 12 November 2010, vol. 5 (11), e13957
- **PALMIERI L. et al.** *Am J Public Health,* 2010, vol. 100 (4), 684-92
- **HOTCHKISS JW et al.** *BMJ,* 06 February 2014, vol. 348, g1088
- **HEVONOJA T. et al.** *Biochim Biophys Acta,* 15 November 2000, vol. 1488 (3), 189-210
- **ROSS R. et al.** *N Engl J Med.,* 14 January 1999, vol. 340 (2), 115-26
- **GURETZKI HJ et al.** *Atherosclerosis,* May 1994, vol. 107 (1), 15-24
- **VÉNIANT MM et al.** *J Clin Invest.,* December 2000, vol. 106 (12), 1501-10
- **LIEU HD et al.** *Circulation,* 11 March 2003, vol. 107 (9), 1315-21
- **WILSON PW et al.** *Circulation,* 12 May 1998, vol. 97 (18), 1837-47
- **SHARRETT AR et al.** *Circulation,* 04 September 2001, vol. 104 (10), 1108-13
- **OTVOS JD et al.** *J Clin Lipidol.,* March 2011, vol. 5 (2), 105-13
- **CARR MC et al.** *J Clin Endocrinol Metab.,* June 2004, vol. 89 (6), 2601-7
- **AUSTIN MA et al.** *JAMA,* 07 October 1988, vol. 260 (13), 1917-21
- **STAMPFER MJ. et al.** *JAMA,* 18 September 1996, vol. 276 (11), 882-8
- **LAMARCHE B. et al.** *Circulation,* 07 January 1997, vol. 95 (1), 69-75
- **TORNVALL P. et al.** *Atherosclerosis,* September 1991, vol. 90 (1), 67-80
- **CAMPOS H. et al.** *Arterioscler Thromb.,* February 1992, vol. 12 (2), 187-95
- **GARDNER CD. et al.** *JAMA,* 18 September 1996, vol. 276 (11), 875-81
- **TABAS I. et al.** *Circulation,* 16 October 2007, vol. 116 (16), 1832-44
- **BERNEIS KK et al.** *J Lipid Res.,* September 2002, vol. 43 (9), 1363-79
- **ANBER V. et al.** *Arterioscler Thromb Vasc Biol.,* November 1997, vol. 17 (11), 2507-14
- **HURT-CAMEJO E. et al.** *Arthritis Rheum.,* December 2001, vol. 44 (12), 2761-7
- **SARTIPY P. et al.** *J Biol Chem.,* 03 September 1999, vol. 274 (36), 25913-20
- **CAMEJO G. et al.** *Atherosclerosis,* August 1998, vol. 139 (2), 205-22
- **AIKAWA M. et al.** *Cardiovasc Pathol.,* May 2004, vol. 13 (3), 125-38
- **MAURIELLO A et al.** *Atherosclerosis,* February 2010, vol. 208 (2), 572-80
- **PURI R. et al.** *Arterioscler Thromb Vasc Biol.,* November 2016, vol. 36 (11), 2220-2228
- **SKÅLÉN K et al.** *Nature,* 13 June 2002, vol. 417 (6890), 750-4
- **OÖRNI K. et al.** *J Lipid Res.,* November 2000, vol. 41 (11), 1703-14
- **OÖRNI K. et al.** *Arterioscler Thromb Vasc Biol.,* August 2005, vol. 25 (8), 1678-83
- **MARATHE S. et al.** *J Biol Chem.,* 13 February 1998, vol. 273 (7), 4081-8
- **SCHISSEL SL et al.** *J Biol Chem.,* 02 August 1996, vol. 271 (31), 18431-6
- **AVIRAM M. et al.** *Biochem Biophys Res Commun.,* 29 May 1992, vol. 185 (1), 465-72
- **OÖRNI K. et al.** *J Biol Chem.,* 30 October 1998, vol. 273 (44), 29127-34
- **HAKALA JK et al.** *Arterioscler Thromb Vasc Biol.,* June 2001, vol. 21 (6), 1053-8
- **TABAS I et al.** *J Biol Chem.,* 25 September 1993, vol. 268 (27), 20419-32

- **SCHISSEL SL et al.** *J Clin Invest.,* 15 September 1996, vol. 98 (6), 1455-64
- **WYLER VON BALLMOOS M. et al.** *Arterioscler Thromb Vasc Biol.,* February 2006, vol. 26 (2), 359-64
- **KHOO JC et al.** *Arteriosclerosis,* July 1988, vol. 8 (4), 348-58
- **ZHANG WY et al.** *J Biol Chem.,* 20 October 2000, vol. 275 (42), 33176-83
- **KRUTH HS.** *Curr Opin Lipidol.,* October 2002, vol. 13 (5), 483-8
- **LLORENTE-CORTÉS V. et al.** *Arterioscler Thromb Vasc Biol.,* May 1998, vol. 18 (5), 738-46, http://atvb.ahajournals.org/content/20/6/1572
- **LLORENTE-CORTÉS V. et al.** *Arterioscler Thromb Vasc Biol.,* 01 March 2002, vol. 22 (3), 387-93
- **LLORENTE-CORTÉS V et al.** *Circulation,* 10 December 2002, vol. 106 (24), 3104-10
- **LLORENTE-CORTÉS V. et al.** *Arterioscler Thromb Vasc Biol.,* May 1998, vol. 18 (5), 738-46
- **LLORENTE-CORTÉS V. et al.** *Circulation,* 10 December 2002, vol. 106 (24), 3104-10
- **COSTALES P. et al.** *Atherosclerosis,* December 2010, vol. 213 (2), 458-68
- **LLORENTE-CORTÉS V. et al.** *J Mol Biol.,* 16 June 2006, vol. 359 (4), 950-60
- **LLORENTE-CORTÉS V. et al.** *Circulation,* 27 July 2004, vol. 110 (4), 452-9
- **CAMINO-LÓPEZ S. et al.** *Cardiovasc Res.,* 01 January 2007, vol. 73 (1), 208-16
- **CAMINO-LÓPEZ S. et al.** *Thromb Haemost.,* December 2009, vol. 7 (12), 2137-46
- **ALLAHVERDIAN S. et al.** *Circulation,* 15 April 2014, vol. 129 (15), 1551-9
- **ETIQUE N. et al.** *Biomed Res Int. 2013,* 2013, 152163
- **LILLIS AP et al.** *J Thromb Haemost.,* August 2005, vol. 3 (8), 1884-93
- **COSTALES P. et al.** *J Biol Chem.,* 12 June 2015, vol. 290 (24), 14852-65
- **DUBROFF R. et al.** *World J Cardiol.,* 26 July 2015, vol. 7 (7), 404-9
- **TOTH PP. et al.** *Am J Cardiovasc Drugs.,* June 2018, vol. 18 (3), 157-173
- **BARYLSKI M. et al.** *Curr Pharm Des.,* 2014, vol. 20 (22), 3657-64
- **PODREZ EA et al.** *Blood,* 10 March 2016, vol. 127 (10), 1221-2
- **BANCELLS C. et al.** *J Lipid Res.,* March 2009, vol. 50 (3), 446-55
- **BANCELLS C. et al.** *Biochemistry,* 05 August 2008, vol. 47 (31), 8186-94
- **BANCELLS C. et al.** *J Biol Chem.,* 15 October 2010, vol. 285 (42), 32425-35
- **IVANOVA EA et al.** *Vasc Health Risk Manag,* 28 August 2015, vol. 11, 525-32
- **MAHLEY RW et al.** *J Lipid Res.,* November 1999, vol. 40 (11), 1933-49
- **SANTOS RD et al.** *Lancet Diabetes Endocrinol.,* October 2016, vol. 4 (10), 850-61
- **STURM AC et al.** *J Am Coll Cardiol.,* 07 August 2018, vol. 72 (6), 662-680
- **MEHTA R et al.** *Atherosclerosis,* October 2018, vol. 277, 204-210
- **MILLÁN J et al.** *Med Clin,* 16 November 2013, vol. 141 (10), 430-6
- **ATHYROS VG et al.** *Hormones,* March 2018, vol. 17 (1), 61-67
- **IQBAL J et al.** *Curr Diabetes Rev.,* 2018, vol. 14 (5), 427-433
- **GERBER PA ; NIKOLIC D ; RIZZO M. SMALL ; DENSE LDL.** *Curr Opin Cardiol.,* July 2017, vol. 32 (4), 454-459
- **SARTIPY P et al.** *J Biol Chem.,* 03 September 1999, vol. 274 (36), 25913-20
- **CAMEJO G et al.** *Atheroscler,* vol. 3 (1), 3-9
- **JAYASINGH IA et al.** *J Family Med Prim Care,* October 2016, vol. 5 (4), 809-816
- **DUNTAS LH et al.** *Front Endocrinol,* 2018, vol. 9, 511
- **FERRAÙ F et al.** *Front Horm Res.,* 2018, vol. 49, 85-103
- **RUUTH M et al.** *Eur Heart Journal,* 2018